(19) Europäisches Patentamt
European Patent Office
Office européen des brevets

(11) Numéro de publication : **0 420 707 B1**

(12) # FASCICULE DE BREVET EUROPEEN

(45) Date de publication du fascicule du brevet : **15.12.93 Bulletin 93/50**

(21) Numéro de dépôt : **90402361.1**

(22) Date de dépôt : **24.08.90**

(51) Int. Cl.$^5$ : **A61K 7/06,** A61K 31/505, C07D 239/42, C07D 239/48, C07D 239/47, C07D 239/84, C07D 239/95, C07D 498/04

(54) **Compositions destinées à être utilisées pour freiner la chute des cheveux et pour induire et stimuler leur croissance, contenant des dérivés de l'amino-2 pyrimidine oxyde-3 et nouveaux composés dérivés de l'amino-2 pyrimidine oxyde-3.**

(30) Priorité : **29.08.89 FR 8911352**

(43) Date de publication de la demande : **03.04.91 Bulletin 91/14**

(45) Mention de la délivrance du brevet : **15.12.93 Bulletin 93/50**

(84) Etats contractants désignés : **AT BE CH DE DK FR GB IT LI NL SE**

(56) Documents cités :
**EP-A- 0 303 871**
**WO-A-86/04231**
**US-A- 3 464 987**
**S. BUDAVARI et al.: "The Merck Index", édition 11, 1989, pages 976-977, résumé no. 6122, Merck & Co., Inc., Rahway, NJ, US**
**CANADIAN JOURNAL OF CHEMISTRY, vol. 62, no. 6, juin 1984, pages 1176-1180, Ottawa, CA; M.V. JOVANOVIC: "Syntheses of some pyrimidine N-oxides"**

(56) Documents cités :
**HELVETICA CHIMICA ACTA, vol. 65, no. 5, mai 1982, pages 1454-1466, Schweizerische Chemische Gesellschaft, Bâle, CH; J.-C. MULLER et al.: "Structure of new 2-oxo-2,8-dihydro-[1,2,4]oxadiazole-[2,3-alphapyrimidinecarbamates"**

(73) Titulaire : **L'OREAL**
**14, Rue Royale**
**F-75008 Paris (FR)**

(72) Inventeur : **Dufetel, Didier**
**82, rue Lavoisier**
**F-77500 Chelles (FR)**
Inventeur : **Estradier, Françoise**
**4, rue Nobel**
**F-75018 Paris (FR)**
Inventeur : **Gaetani, Quintino**
**64, avenue Hoche**
**F-93270 Sevran (FR)**
Inventeur : **Hocquaux, Michel**
**70, rue du Rendez-vous**
**F-75012 Paris (FR)**

(74) Mandataire : **Casalonga, Axel et al**
**BUREAU D.A. CASALONGA - JOSSE**
**Morassistrasse 8**
**D-80469 München (DE)**

EP 0 420 707 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne des compositions destinées à être utilisées, notamment en application topique, pour freiner la chute des cheveux et pour induire et stimuler leur croissance, contenant des dérivés de l'amino-2 pyrimidine oxyde-3 ainsi que des nouveaux dérivés de l'amino-2 pyrimidine oxyde-3 utilisés dans ces compositions.

On connaît déjà, dans l'état de la technique, le diamino-2,4 pipéridino-6 pyrimidine oxyde-3 ou "Minoxidil" pour ses propriétés d'agent anti-hypertenseur, mais également pour son utilisation dans le traitement de la chute des cheveux, de la pelade, de la dermatite desquamante et de l'alopécie.

On connaît également les composés amino-7 (amino-substitué-5)-2H-oxadiazolo-1,2,4 [2,3a] pyrimidinone et leur forme isomère amino-7 (aminosubstitué-5)-2H-1,2,4- [2,3c] - pyrimidinone-2 pour leur utilisation dans le traitement de la chute des cheveux et des maladies du cuir chevelu. Ils sont décrits dans la demande de brevet EP 0 303 871.

La demanderesse a découvert de nouvelles compositions pour le traitement et la prévention de la chute des cheveux, utilisées notamment en application topique, particulièrement efficaces dans le traitement des maladies du cuir chevelu grâce à une famille particulière de composés dérivés de l'amino-2 pyrimidine oxyde-3.

Les composés retenus par la demanderesse, en plus du fait qu'ils sont efficaces pour la repousse des cheveux et notamment pour induire et stimuler leur croissance et freiner leur chute, présentent une activité anti-hypertensive sensiblement nulle ou plus faible que celle du Minoxidil.

Ces composés présentent des solubilités dans les milieux habituellement utilisés en cosmétique et en pharmacie nettement supérieures à celles du Minoxidil.

L'invention a donc pour objet de nouvelles compositions destinées au traitement et à la prévention de la chute des cheveux, contenant des composés particuliers dérivés de l'amino-2 pyrimidine oxyde-3.

L'invention a également pour objet de nouveaux dérivés de l'amino-2 pyrimidine oxyde-3 utilisés dans ces compositions.

Un autre objet concerne l'utilisation des composés selon l'invention pour la préparation d'un médicament destiné au traitement thérapeutique de la chute des cheveux.

D'autres objets de l'invention apparaîtront à la lecture de la description et des exemples qui suivent.

Les compositions conformes à l'invention sont essentiellement caractérisées par le fait qu'elles contiennent dans un milieu physiologiquement acceptable, au moins un composé répondant à la formule suivante :

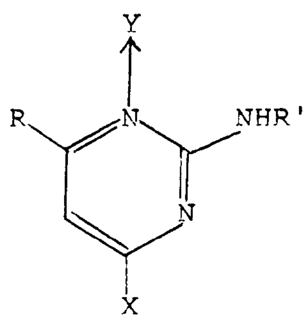

(I)

dans laquelle :

R désigne un atome d'hydrogène, un radical alkyle, saturé et linéaire, en $C_1$-$C_8$ ou forme avec le cycle pyrimidine , un cycle hydrocarboné de formule :

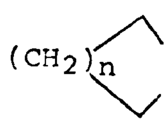

avec n égal à 1, 2 ou 3;

X désigne :

(i) un atome d'hydrogène;

(ii) un groupement

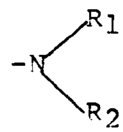

dans lequel :

$R_1$ et $R_2$, identiques ou différents, désignent un atome d'hydrogène, un groupe alkyle saturé en $C_1$-$C_{12}$, linéaire ou ramifié, pouvant être substitué par un atome d'halogène ou un radical trifluorométhyle, un groupe alcényle linéaire en $C_2$-$C_{12}$, un groupe cycloalkyle en $C_3$-$C_{10}$, un groupe aralkyle en $C_7$-$C_{12}$ ou un groupement aryle répondant à la formule :

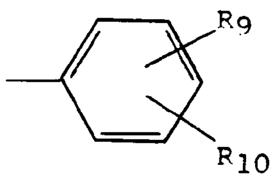

dans laquelle $R_9$ et $R_{10}$, identiques ou différents, désignent hydrogène, alkyle en $C_1$-$C_4$, hydroxyle, alcoxy en $C_1$-$C_4$ ou halogène;

$R_1$ et $R_2$ peuvent former avec l'atome d'azote auquel ils sont reliés, un hétérocycle saturé ou insaturé, choisi parmi les groupes suivants : aziridino, azétidino, pyrrolidino, pipéridino, hexaméthylèneimino, heptaméthylèneimino, octaméthylèneimino, tétrahydropyridino, dihydropyridino, pyrrole, pyrrazole, imidazole, triazole, alkyl-4 pipérazino, morpholino, thiomorpholino;

(iii) un groupement $-OR_3$, dans lequel $R_3$ désigne un radical alkyle saturé en $C_1$-$C_{12}$, linéaire ou ramifié, pouvant être substitué par un atome d'halogène ou un radical trifluorométhyle, un radical alcényle linéaire en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'ature, désignent un radical alkyle en $C_1$-$C_6$, un radical alcoxy en $C_1$-$C_6$, un atome d'halogène ou un radical trifluorométhyle;

(iv) un groupement $-SR_4$, dans lequel $R_4$ a la même signification que $R_3$ définie ci-dessus;

Y désigne un atome d'oxygène ou un groupement $-OSO_3^{\ominus}$ ;

R' désigne un atome d'hydrogène ou l'un des groupements suivants :

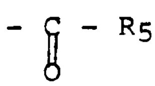 , 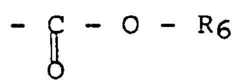 , 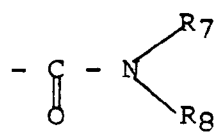

dans lesquels :

$R_5$ et $R_6$ représentent des radicaux alkyle inférieurs en $C_1$-$C_4$;

$R_7$ et $R_6$ désignent un atome d'hydrogène ou un radical alkyle inférieur en $C_1$-$C_4$, à condition qu'ils ne désignent pas simultanément un atome d'hydrogène.

Lorsque Y désigne un atome d'oxygène, les composés de formule (I), conformes à l'invention, peuvent coexister avec leur forme tautomère de formule ($I_2$) selon l'équilibre suivant :

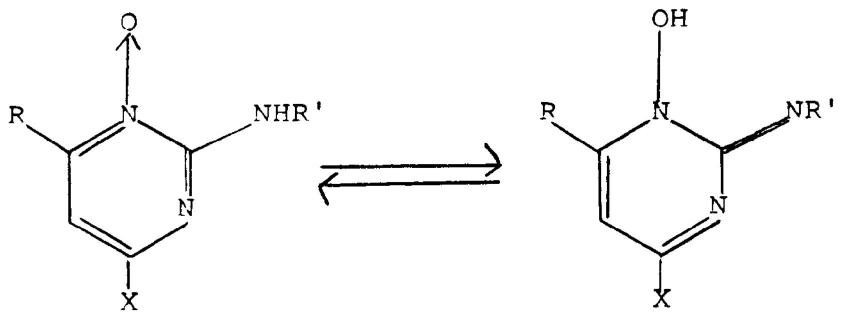

(I) (I2)

Selon la nature du milieu, l'une des formes tautomères peut être prépondérante par rapport à l'autre.

Les composés de formule (I), conformes à l'invention, peuvent être transformés en leurs sels d'addition d'acides cosmétiquement ou pharmaceutiquement acceptables, tels que les sels des acides sulfurique, chlorhydrique, bromhydrique, phosphorique, acétique, benzoïque, salicyclique, glycolique, succinique, nicotinique, tartrique, maléique, pamoïque, méthane sulfonique, picrique, lactique, etc.

Parmi les composés de formule générale (I), un certain nombre de composés sont connus en eux-mêmes et ont été décrits comme agents antihypertenseurs ou comme intermédiaires de synthèse.

Ils sont notamment décrits dans les brevets américains USP 3 464 987, 3 644 364, 4 013 778 et 4 287 338; le brevet français n° 2 087 936; les brevets européens EP 058 476 et EP 057 546 ou cités dans la littérature technique (Can J. Chem. 1984, 62(6), 1176-80), (Müller, Ramuz, Helv. Chim., Act. 65 (5), 1982, pages 1454-66).

Des composés nouveaux conformes à la présente invention, répondent à la formule (I') suivante :

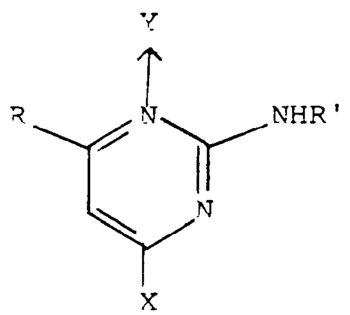

(I')

dans laquelle :

R, R' et Y ont les mêmes significations indiquées dans la formule (I) ci-dessus et X désigne :

(i) un groupement -$SR_4$, dans lequel $R_4$ désigne un radical alkyle saturé en $C_1$-$C_{12}$, linéaire ou ramifié, pouvant être substitué par un atome d'halogène ou un radical trifluorométhyle, un radical alcényle linéaire en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un atome d'halogène, un radical alkyle en $C_1$-$C_6$, un radical alcoxy en $C_1$-$C_6$ ou un radical trifluorométhyle;

(ii) un groupement -$OR_3$, dans lequel $R_3$ a la même signification que $R_4$, à la condition que lorsque Y désigne un atome d'oxygène, R un groupement alkyle et R' désigne un atome d'hydrogène, $R_3$ ne désigne pas un radical alkyle en $C_1$-$C_4$ ou un radical phényle, éventuellement substitué par un ou deux atomes d'halogène.

Les composés nouveaux de formule (I') peuvent se trouver sous forme de sels d'addition d'acides physiologiquement acceptables.

Les composés conformes à la présente invention répondant à la formule générale (I), sont obtenus à partir d'un dérivé amino-2 pyrimidine oxyde-3 substitué en position 6, de formule suivante :

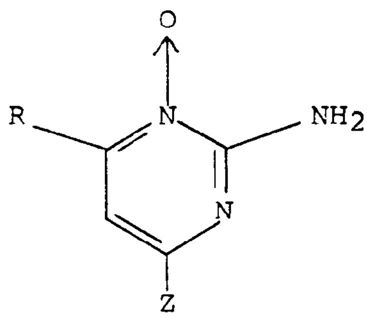

(II)

dans laquelle :

R a la signification indiquée dans la formule générale (I);

Z désigne un atome d'halogène choisi parmi le chlore ou le brome, un groupement sulfonate tel que tosylate, brosylate ou mésylate ou un groupement phénoxy substitué par des groupes électro-attracteurs, comme les atomes d'halogène ou des groupements nitro.

Les composés particuliers, selon l'invention, répondant à la formule (IA) :

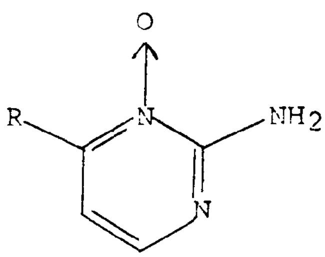

(IA)

dans laquelle R a la signification indiquée dans la formule (I) ci-dessus, sont obtenus par hydrogénolyse des composés de formule (II), dans laquelle Z désigne plus particulièrement un atome de chlore ou de brome. La réduction est effectuée suivant les méthodes classiques décrites dans la littérature technique (D.J. Brown, The pyrimidines, Vol. 16, supplement II, Chapitre X, 360). Leur procédé de préparation peut être représenté par le schéma suivant :

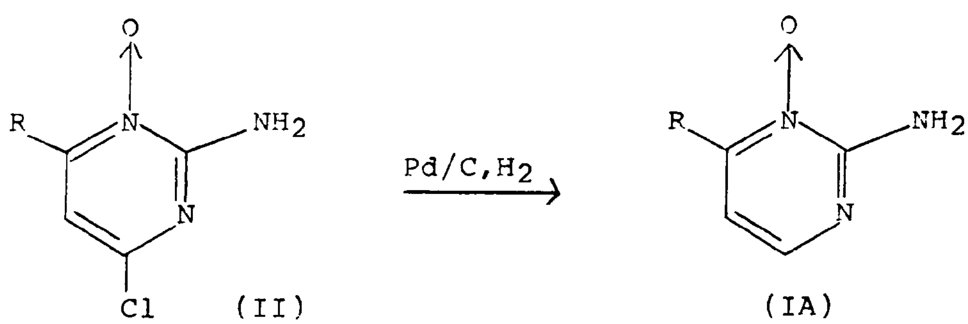


(II) (IA)

SCHEMA A

Les composés particuliers de formule (IB) :

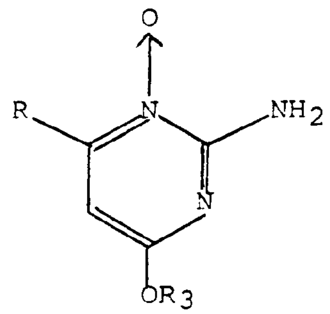

(IB)

sont obtenus en faisant réagir une solution de l'alcoolate $R_3O^{\ominus}$ $W^{\oplus}$, dans laquelle $R_3$ a la même signification que celle indiquée pour la formule générale (I) et W désigne un métal alcalin tel que le sodium, le potassium ou le lithium, dans l'alcool correspondant, sur les composés de formule (II) dans laquelle Z désigne le chlore ou le brome, ou un groupement phénoxy substitué par des groupes électro-attracteurs.

On applique la méthode de Williamson telle que décrite dans le brevet européen EP-57 546, à une température comprise entre 40 et 100°C.

La préparation de ces composés peut être représentée par le schéma suivant :

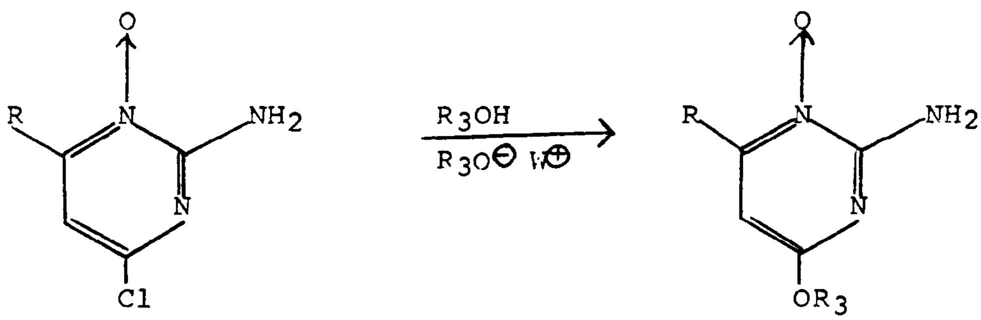


(II) (IB)

SCHEMA B

Les composés particuliers de formule (IC) suivante :

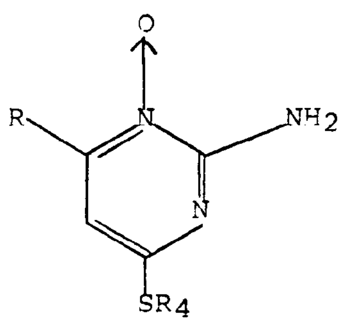

(IC)

sont préparés en faisant réagir sur les composés de formule (II) un thiolate de formule $R_4S^{\ominus}$ $W^{\oplus}$, dans laquelle $R_4$ et W ont les significations indiquées précédemment, en présence d'un solvant choisi parmi les éthers, de préférence le Méthylcellosolve ou le diméthyléther de l'éthylèneglycol, à une température de l'ordre de 50 à 150°C.

On procède suivant les méthodes classiques de la littérature (D.J. Brown, the PYRIMIDINES, Vol. 16, Supplement II, Chapitre VI, Section F).

La préparation de ces composés peut être représentée par le schéma suivant :

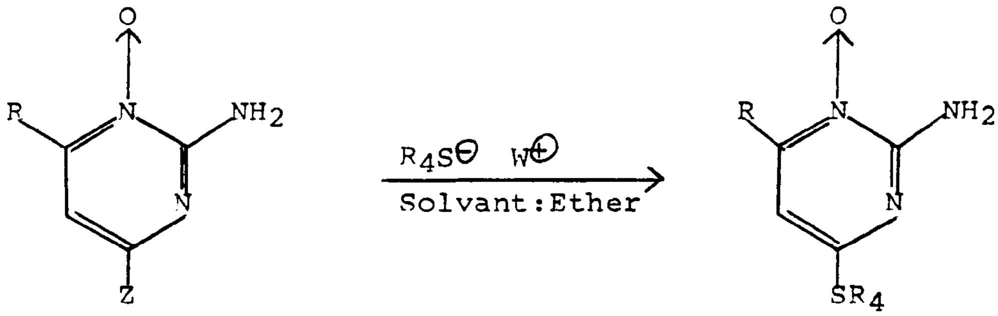


(II) (IC)

SCHEMA C

Les composés particuliers de formule (ID) suivante :

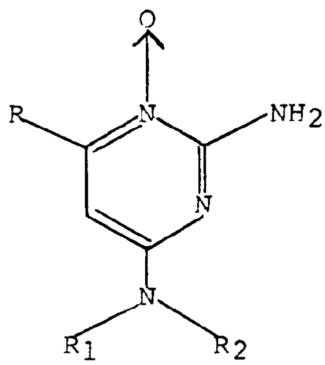

(ID)

sont obtenus en faisant réagir une amine

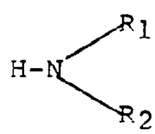

dans laquelle $R_1$ et $R_2$ ont les mêmes significations que celles indiquées pour la formule générale (I), sur les composés de formule (II). On procède en présence d'un solvant qui peut être un alcool, de préférence l'éthanol, ou l'amine servant de réactif et à la fois de solvant, à une température comprise entre 20 et 150°C, suivant les procédés décrits dans les brevets américains USP 3 644 364 et 3 464 987.

La préparation de ces composés peut être représentée par le schéma suivant :

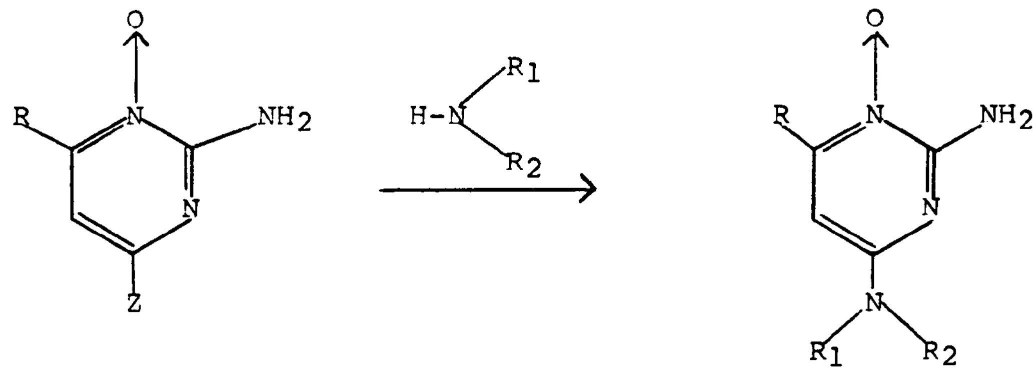

(II) (ID)

SCHEMA D

Les composés particuliers selon l'invention de formule (I), dans laquelle Y désigne un atome d'oxygène, obtenus selon les différents procédés décrits précédemment, peuvent être transformés en leurs homologues O-sulfates, par sulfatation chimique, selon les méthodes classiques décrites dans la littérature (J. Med. Chem. 1983, 26, p. 1791-1793).

On utilise comme réactif de sulfatation les complexes de trioxyde de soufre-pyridine, de trioxyde de soufre-triéthylamine ou de trioxyde de soufre-éthyl diisopropylamine.

Les solvants utilisés sont de préférence le diméthylformamide, l'acétonitrile, le chloroforme ou leurs mélanges binaires. La température est de l'ordre de 0 à 25°C et le temps de réaction varie entre 1 heure et 24 heures.

Les composés particuliers, selon l'invention, de formule (I) dans laquelle Y désigne un atome d'oxygène, peuvent être transformés en leurs homologues amides, uréides ou carbamates, suivant les méthodes classiques décrites dans la littérature (J. MARCH, Advanced Organic Chemistry, 3ème édition, p. 370) par action respectivement d'un chlorure d'acide, d'un anhydride d'acide, d'un chlorure d'acide carbamique ou d'un chloroformiate d'alkyle; on procède en présence d'une amine tertiaire, telle que la pyridine.

Les composés ainsi obtenus, de formule :

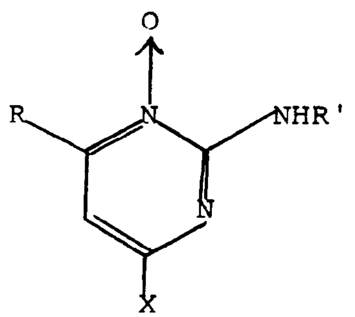

(IE)

dans laquelle R, R', X ont la même signification que celle indiquée pour la formule (I) définie ci-dessus, sont facilement hydrolysables, en milieu potasse alcoolique, par exemple, et peuvent redonner naissance à leurs précurseurs de formule (I), dans laquelle R' désigne un hydrogène.

La transformation des composés de formule (I) dans laquelle Y est l'oxygène et R' est l'hydrogène, en leurs homologues O-sulfates, amides, uréides ou carbamates, peut être représentée par le schéma suivant :

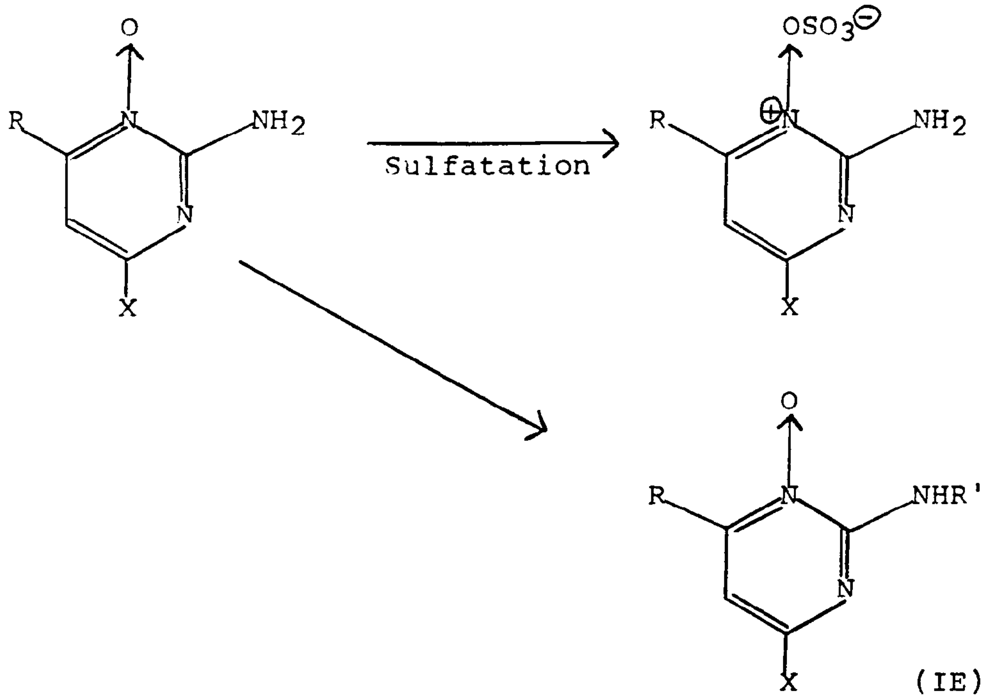


(IE)

SCHEMA E

Les composés de formule (IE), conformes à l'invention, peuvent constituer des intermédiaires pour la synthèse des oxadiazolopyrimidines de formule (III) suivante :

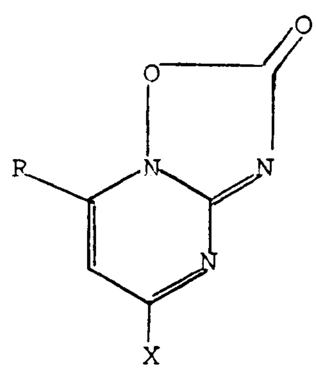

(III)

dans laquelle R et X ont la signification indiquée dans la formule générale (I).

Les composés (III) sont obtenus par cyclisation-élimination interne des dérivés carbamates ou uréides de formule (IE) selon les méthodes décrites dans la littérature (J.C. MÜLLER, Helvetica Chimica Acta, Vol. 66, 1983, p. 669-672).

Les composés de formule (III) et leurs sels sont nouveaux, à l'exception du composé suivant : 2-oxo-2,8-dihydro[1,2,4]oxadiazolo[2,3-a]pyrimidine carbamate, qui est décrit dans le document (MÜLLER, RAMUZ, Helvetica Chimica Acta 65 (5), 1982, p. 1454-66) et constituent un autre objet de l'invention. Ils peuvent recevoir des applications diverses et notamment dans l'utilisation pour le traitement et la prévention de la chute des cheveux.

Les compositions conformes à la présente invention, contenant dans un milieu physiologiquement acceptable, au moins un composé répondant à la formule (I) ou un de ses sels d'addition d'acides physiologiquement acceptables, peuvent être appliquées dans le domaine cosmétique ou pharmaceutique, notamment en application topique. Elles sont destinées pour le traitement et la prévention de la chute des cheveux et notamment de la pelade, de l'alopécie ainsi que des dermatites desquamantes.

Ces compositions peuvent comporter, à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées en pharmacie se présentent sous forme d'onguent, de teinture, de crème, de pommade, de poudre, de timbre, de tampon imbibé, de solution, d'émulsion ou de dispersion vésiculaire, de lotion, de gel, de spray ou de suspension. Elles peuvent être, soit anhydres, soit aqueuses, selon l'indication clinique.

Les composés selon l'invention sont présents dans ces compositions pharmaceutiques à des concentrations comprises entre 0,1 et 10% en poids, et en particulier comprises entre 0,2 et 5% en poids.

Les compositions cosmétiques sont notamment destinées à être utilisées sous forme de lotion, de gel, de savon, de shampooing, d'aérosol ou de mousse et contiennent, dans un support cosmétiquement acceptable, au moins un composé de formule (I) ou l'un de ses sels d'addition d'acides.

La concentration de ces composés de formule (I) dans ces compositions est, de préférence, comprise entre 0,01 et 5% en poids et en particulier entre 0,05 et 3% en poids.

Les compositions conformes à l'invention peuvent contenir différents additifs habituellement utilisés en cosmétique ou en pharmacie et en particulier des substances actives, telles que des agents hydratants comme la thiamorpholinone et ses dérivés ou l'urée; des agents antiséborrhéiques, tels que la S-carboxyméthylcystéine, la S-benzylcystéamine, et leurs dérivés; la thioxolone.

Les composés conformes à l'invention peuvent être associés à des composés améliorant encore leur activité sur la repousse et/ou sur le freinage de la chute des cheveux, tels que plus particulièrement les composés suivants :

- les esters d'acide nicotinique, dont plus particulièrement les nicotinates d'alkyle en $C_1$-$C_6$ et notamment le nicotinate de méthyle;
- les agents anti-inflammatoires stéroïdiens et non stéroïdiens bien connus dans l'état de la technique et en particulier l'hydrocortisone, ses sels et ses dérivés, l'acide niflumique;
- les rétinoïdes et plus particulièrement l'acide t-trans rétinoïque appelé encore trétinoïne, l'isotrétinoïne, le rétinol ou la vitamine A et ses dérivés, tels que l'acétate, le palmitate ou le propionate, le motrétinide, l'étrétinate, le t-trans rétinoate de zinc;
- les agents antibactériens choisis plus particulièrement parmi les macrolides, les pyranosides et les tétracyclines et notamment l'érythromycine;
- les agents antagonistes de calcium, tels que plus particulièrement la cinnarizine et le diltiazem;
- des hormones, telles que l'estriol ou des analogues ou la thyroxine et ses sels;
- des agents antiandrogènes, tels que l'oxendolone, la spironolactone, le diéthylstilbestrol;
- des capteurs de radicaux OH, tels que le diméthylsulfoxyde.

On peut également associer avec les composés de l'invention, éventuellement en mélange avec les autres, des composés tels que le Diazoxyde correspondant au méthyl-3 chloro-7 2H benzothiadiazine 1,2,4-dioxyde-1,1; la Spiroxasone ou 7-(acétylthio)-4',5'-dihydrospiro[androst 4-ène-17,2'-(3'H)furan]-3 one; des phospholipides, tels que la lécithine; les acides linoléique et linolénique; l'acide salicylique et ses dérivés décrits plus particulièrement dans le brevet français 2 581 542, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters, des lactones et leurs sels correspondants; l'anthraline ou la trihydroxy-1,8,9 anthracène, les caroténoïdes, les acides eicosatétraynoïque-5,8,11,14 ou eicosatriynoïque-5,8,11, leurs esters et amides.

Les composés conformes à l'invention peuvent également être associés à des agents tension-actifs dont plus particulièrement ceux choisis parmi les agents tension-actifs non ioniques et amphotères.

Parmi les tensio-actifs non ioniques, on citera plus particulièrement les polyhydroxypropyléthers décrits notamment dans les brevets français n° 1 477 048; 2 091 516; 2 169 787; 2 328 763; 2 574 786; les alkyl($C_8$-$C_9$)phénols oxyéthylénés comportant de 1 à 100 moles d'oxyde d'éthylène et de préférence 5 à 35 moles d'oxyde d'éthylène; les alkyl polyglycosides de formule :

$$C_nH_{2n+1}(C_6H_{10}O_5)_xH \qquad (A)$$

dans laquelle n varie de 8 à 15 inclus et x de 1 à 10 inclus.

Parmi les agents tensio-actifs amphotères, on citera plus particulièrement les amphocarboxyglycinates et les amphocarboxypropionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléther d'alkylèneglycol, en particulier d'alkyle en $C_1$-$C_4$, éther d'alkylène en $C_1$-$C_4$ glycol ou de dialkylèneglycol, de préférence de dialkylène en $C_1$-$C_4$ glycol, d'alcool éthylique et

d'eau, le solvant glycolique désignant plus particulièrement le monoéthyléther de l'éthylène glycol, le monométhyléther du propylèneglycol, le monométhyléther du diéthylèneglycol.

Les composés conformes à l'invention peuvent également être introduits dans des supports gélifiés ou épaissis, tels que des supports essentiellement aqueux gélifiés par des hétérobiopolysaccharides, tels que la gomme de xanthane ou les dérivés de cellulose, des supports hydroalcooliques gélifiés par des polyhydroxy éthylacrylates ou méthacrylates ou des supports essentiellement aqueux épaissis en particulier par des acides polyacryliques réticulés par un agent polyfonctionnel, tel que les Carbopol vendus par la Société GOODRICH.

Ces compositions peuvent également contenir des agents conservateurs, des agents stabilisants, des agents régulateurs de pH, des agents modificateurs de pression osmotique, des agents émulsifiants, des filtres UVA et UVB, des agents antioxydants, tels que 1' $\alpha$-tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique et choisis plus particulièrement parmi les alcools inférieurs en $C_1$-$C_4$, comme l'alcool éthylique, l'alcool isopropylique, l'alcool tertiobutylique, les alkylèneglycols, les alkyléthers d'alkylèneglycol et de dialkylèneglycol, tels que le monoéthyléther d'éthylèneglycol, le monométhyléther de propylèneglycol, le monométhyléther de diéthylèneglycol. Les solvants, lorsqu'ils sont présents, le sont dans des proportions comprises entre 1 et 80% en poids par rapport au poids total de la composition.

Les milieux physiologiquement acceptables peuvent être épaissis à l'aide d'agents épaississants habituellement utilisés en cosmétique ou pharmacie, et on peut plus particulièrement citer les hétérobiopolysaccharides tels que la gomme de xanthane, les scléroglucanes, les dérivés de cellulose comme les éthers de cellulose, les polymères acryliques, réticulés ou non.

Les épaississants sont présents de préférence dans des proportions comprises entre 0,1 et 5% en poids et en particulier entre 0,4 et 3% en poids par rapport au poids total de la composition.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Un autre objet de l'invention est constitué par l'utilisation de la composition contenant les composés de formule (I) définie ci-dessus, pour la préparation d'un médicament ayant pour effet d'induire ou de stimuler la croissance des cheveux et de freiner leur chute.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant un durée de 1 à 6 mois.

Les compositions peuvent notamment être utilisées dans le traitement de la pelade, de la chute des cheveux, des dermatites desquamantes.

Les exemples suivants sont destinés à illustrer l'invention sans pour autant présenter un caractère limitatif.

## EXEMPLES DE PREPARATION

### EXEMPLE 1

Amino-2 méthyl-4 pipéridino-6 pyrimidine oxyde-3

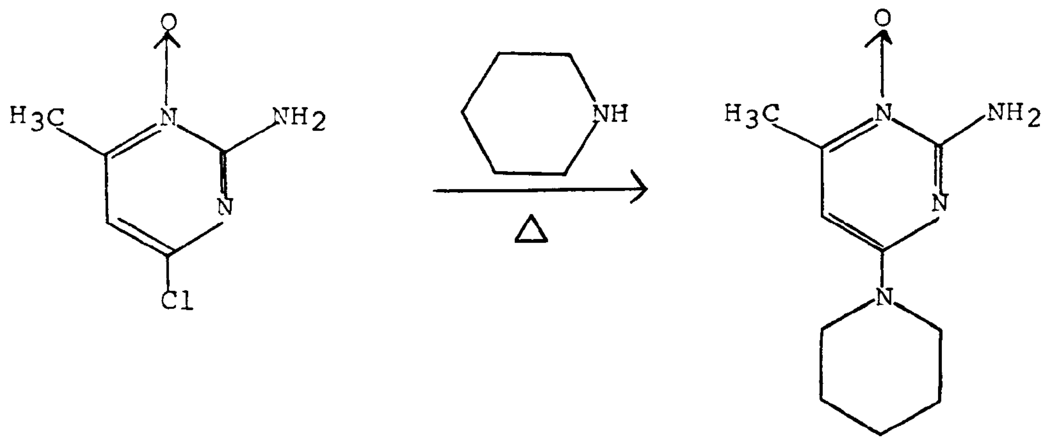

Mode opératoire :

15 g (9,35.10$^{-2}$mole) d'amino-2 méthyl-4 chloro-6 pyrimidine oxyde-3 sont introduits en suspension dans 150 ml de pipéridine. Le mélange est chauffé à 100°C pendant 8 heures. Après refroidissement du milieu réactionnel, le précipité est filtré, lavé avec de la pipéridine puis l'éther éthylique. Il est ensuite ajouté à 35 ml d'une solution aqueuse de soude à 10% contenant 6 g de chlorure de sodium; on laisse agiter 1/2 heure puis on filtre le précipité résultant qui, après séchage, est recristallisé dans le système eau-éthanol (80/20).

Masse obtenue = 12,5 g
Rendement = 64%.
P.F. = 262°C.

Analyse élémentaire pour $C_{10}H_{16}N_4O$ ; PM = 208.

| | C | H | N | O |
|---|---|---|---|---|
| Calculé | 57,69 | 7,69 | 26,92 | 7,69 |
| Trouvé | 57,76 | 7,73 | 26,81 | 7,71 |

Les spectres de RMN $^1$H et $^{13}$C ainsi que le spectre de masse sont conformes à la structure.

### EXEMPLE 2

Amino-2 méthyl-4 pyrrolidino-6 pyrimidine oxyde-3

Mode opératoire :

Dans un tricol de 250 ml muni d'un agitateur magnétique, d'un thermomètre et d'un réfrigérant, on place 9 g (5,61.10$^{-2}$mole) d'amino-2 méthyl-4 chloro-6 pyrimidine oxyde-3 en suspension dans 90 ml de pyrrolidine. On agite le milieu réactionnel pendant 2 heures à température ambiante, soit 25°C. Après avoir refroidi à 5°C, on filtre le précipité sur verre fritté. On le lave par 100 ml d'acétone puis par 2x100 ml d'éther éthylique. Masse obtenue = 8,85 g.

Le précipité est recristallisé dans un mélange acétonitrile-eau (95/5). On obtient un précipité en paillettes blanches.

Masse obtenue = 4,70 g
Rendement = 43%.
P.F. = 260°C (décomposition).

Analyse élémentaire pour $C_9H_{14}N_4O$ ; PM = 194

| | C | H | N | O |
|---|---|---|---|---|
| Calculé | 55,67 | 7,22 | 28,86 | 8,25 |
| Trouvé | 55,69 | 7,29 | 29,03 | 8,37 |

Les spectres de RMN $^{13}C$ et de masse sont conformes à la structure.

EXEMPLE 3

Amino-2 méthyl-4 morpholino-6 pyrimidine oxyde-3

Mode opératoire :

Dans un tricol de 100 ml muni d'un agitateur magnétique, d'un thermomètre et d'un réfrigérant, on place 5 g (3,12.$10^{-2}$mole) d'amino-2 méthyl-4 chloro-6 pyrimidine oxyde-3 en suspension dans 50 ml de morpholine. On agite le milieu réactionnel pendant 2 heures à température ambiante, soit 25°C. Après avoir refroidi à 5°C, on filtre le précipité sur verre fritté. On le lave par 2x50 ml d'acétone puis par 2x50 ml d'éther éthylique. Masse obtenue = 9,10 g.

On reprend le précipité dans 90 ml de potasse alcoolique à 10%. On agite 1 heure puis on filtre sur un verre fritté préalablement rempli de silice. La solution est évaporée à sec.

Le précipité est recristallisé dans un mélange acétonitrile-eau (90/10).

Masse obtenue = 2,1 g.
Rendement = 35%.
P.F. > 260°C.

Analyse élémentaire pour $C_9H_{14}N_4O_2$ ; PM = 210

| | C | H | N | O |
|---|---|---|---|---|
| Calculé | 51,43 | 6,66 | 26,67 | 15,24 |
| Trouvé | 51,48 | 6,71 | 26,57 | 15,48 |

Les spectres de RMN $^{13}C$ et de masse sont conformes à la structure.

EXEMPLE 4

Amino-2 méthyl-4 thiomorpholino-6 pyrimidine oxyde-3

On suit le mode opératoire décrit à l'exemple 3, mais en utilisant la thiomorpholine.

Température : 25°C
Temps : 2 heures
Recristallisation dans acétonitrile-éthanol (90/10)
Rendement = 42%.
P.F. = 243°C.

Analyse élémentaire pour $C_9H_{14}N_4OS$ ; PM = 226

| | C | H | N | O | S |
|---|---|---|---|---|---|
| Calculé | 47,78 | 6,19 | 24,78 | 7,08 | 14,16 |
| Trouvé | 47,88 | 6,25 | 24,86 | 7,22 | 14,03 |

Les spectres de RMN $^{13}C$ et de masse sont conformes à la structure.

EXEMPLE 5

Amino-2 méthyl-4 (N-méthylpipérazino)-6 pyrimidine oxyde-3

On suit le mode opératoire décrit à l'exemple 3, en utilisant la N-méthylpipérazine.

Température : 30°C
Temps : 18 heures
Recristallisation dans le système acétonitrile-éthanol (50/50)
Rendement = 36%
P.F. = 220°C.

Analyse élémentaire pour $C_{10}H_{17}N_5O.1/4H_2O$ ; PM = 223

| | C | H | N | O |
|---|---|---|---|---|
| Calculé | 52,75 | 7,69 | 30,77 | 8,79 |
| Trouvé | 52,90 | 7,66 | 30,95 | 8,83 |

Calculé avec 0,25 mol $H_2O$.

Les spectres de RMN $^{13}C$ et de masse sont conformes à la structure.

EXEMPLE 6

Amino-2 méthyl-4 diéthylamino-6 pyrimidine oxyde-3

Mode opératoire :

Dans un tricol de 250 ml muni d'un agitateur magnétique, d'un thermomètre et d'un réfrigérant, on place 10 g (6,24.10$^{-2}$mole) d'amino-2 méthyl-4 chloro-6 pyrimidine oxyde-3 en suspension dans 70 ml d'éthanol. On ajoute à température ambiante 10 équivalents de diéthylamine, soit 60 ml. Le milieu réactionnel est chauffé à 50°C pendant 24 heures. On refroidit à 5°C puis on ajoute 70 ml de potasse alcoolique à 10%.

On agite 1 heure. On filtre sur un verre fritté préalablement rempli de silice. La solution est évaporée à sec. Masse obtenue = 9,5 g.

Le précipité est recristallisé dans l'acétonitrile. Masse = 7,15 g.

Rendement = 58%
P.F. = 188°C.

Analyse élémentaire pour $C_9H_{16}N_4O$ ; PM = 196

| | C | H | N | O |
|---|---|---|---|---|
| Calculé | 55,10 | 8,16 | 28,57 | 8,16 |
| Trouvé | 54,93 | 8,13 | 28,77 | 8,40 |

Les spectres de RMN $^{13}C$ et de masse sont conformes à la structure.

EXEMPLE 7

Amino-2 méthyl-4 diméthylamino-6 pyrimidine oxyde-3

On suit le mode opératoire décrit à l'exemple 6, en utilisant la diméthylamine.

Température : 25°C

Temps : 3 heures
Recristallisation dans le système acétonitrile-eau (90/10)
Rendement = 67%
P.F. = 222°C

Analyse élémentaire pour $C_7H_{12}N_4O$ ; PM = 168

| | C | H | N | O |
|---|---|---|---|---|
| Calculé | 50,00 | 7,14 | 33,33 | 9,52 |
| Trouvé | 49,99 | 7,16 | 33,29 | 9,64 |

Les spectres de RMN $^{13}C$, $^{1}H$ et de masse sont conformes à la structure.

EXEMPLE 8

Amino-2 méthyl-4 diallylamino-6 pyrimidine oxyde-3

Mode opératoire :

Dans un tricol de 100 ml muni d'un agitateur magnétique, d'un thermomètre et d'un réfrigérant, on place 5 g ($3,12.10^{-2}$mole) d'amino-2 méthyl-4 chloro-6 pyrimidine oxyde-3 en suspension dans 50 ml d'éthanol. On ajoute à température ambiante 2,2 équivalents de diallylamine, soit 6,7 g. Le milieu réactionnel est chauffé à 60°C pendant 24 heures.

On refroidit à 5°C puis on filtre le précipité correspondant au reste de l'amino-2 méthyl-4 chloro-6 pyrimidine oxyde-3. La solution est évaporée à sec. On additionne de l'éthanol chlorhydrique jusqu'à pH acide. Par addition d'éther éthylique, on précipite le monochlorhydrate de l'amino-2 méthyl-4 diallylamino-6 pyrimidine oxyde-3.

Ce précipité est recristallisé dans un mélange acétone/éthanol.
Masse obtenue = 1 g
Rendement = 12%
PF = 166°C
Le dérivé est isolé sous forme de son chlorhydrate.

Analyse élémentaire pour $C_{11}H_{17}N_4OCl$ ; PM = 256,5

| | C | H | N | O | Cl |
|---|---|---|---|---|---|
| Calculé | 51,45 | 6,63 | 21,83 | 6,23 | 13,84 |
| Trouvé | 51,56 | 6,65 | 21,92 | 6,38 | 13,72 |

Les spectres de RMN et de masse sont conformes à la structure.

EXEMPLE 9

Amino-2 méthyl-4(n-butylamino)-6 pyrimidine oxyde-3

On suit le mode opératoire décrit dans l'exemple 2, en utilisant la n-butylamine.
Température : 55°C
Temps : 18 heures
Recristallisation dans le système acétonitrile-eau (70/30)
Rendement = 49%
P.F. = 216°C.

Analyse élémentaire pour $C_9H_{16}N_4O$ ; PM = 196

| | C | H | N | O |
|---|---|---|---|---|
| Calculé | 55,10 | 8,16 | 28,57 | 8,16 |
| Trouvé | 54,91 | 8,22 | 28,72 | 8,25 |

Les spectres de RMN $^1H$ et de masse sont conformes à la structure.

EXEMPLE 10

Amino-2 méthyl-4 benzylamino-6 pyrimidine oxyde-3

On suit le mode opératoire décrit dans l'exemple 2, en utilisant la benzylamine.

Température : 60°C
Temps : 6 heures
Recristallisation dans le système eau-éthanol (75/25)
Rendement = 53%
P.F. = 226°C

Analyse élémentaire pour $C_{12}H_{14}N_4O$ ; PM = 230

| | C | H | N | O |
|---|---|---|---|---|
| Calculé | 62,60 | 6,09 | 24,35 | 6,95 |
| Trouvé | 62,69 | 6,02 | 24,43 | 7,06 |

Les spectres de RMN $^1H$ et de masse sont conformes à la structure.

EXEMPLE 11

Ethoxycarbonylamino-2 méthyl-4 pipéridino-6 pyrimidine oxyde-3

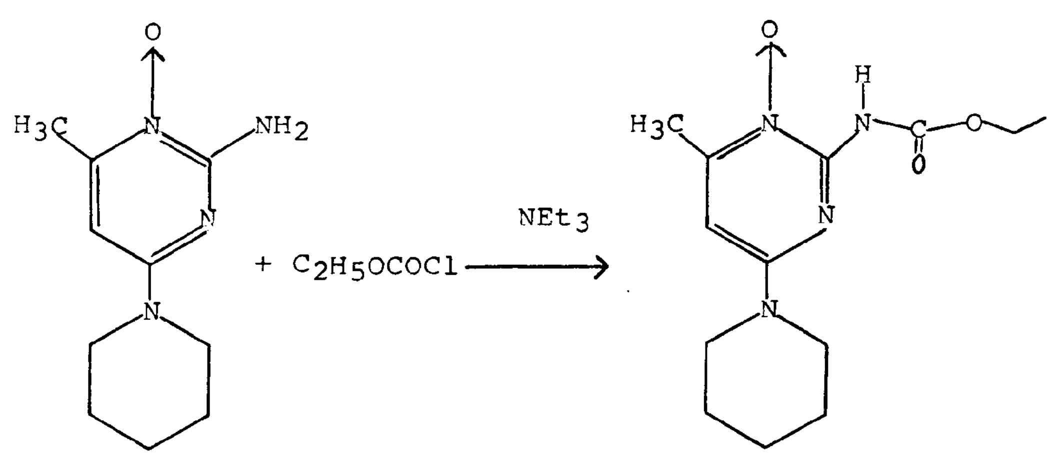


Par action du chloroformiate d'éthyle sur l'amino-2 méthyl-4 pipéridino-6 pyrimidine oxyde-3, en présence de triéthylamine, on obtient le carbamate d'éthyle attendu.

Mode opératoire :

Dans un tricol de 50 ml muni d'un agitateur magnétique, d'un thermomètre et d'une ampoule à addition, on place 2 g (1,25.$10^{-2}$mole) d'amino-2 méthyl-4 pipéridino-6 pyrimidine oxyde-3 en suspension dans 20 ml de dichlorométhane préalablement séché sur tamis moléculaire. On ajoute 1 équivalent de triéthylamine, soit 1,35 ml. On refroidit le milieu réactionnel à 0°C et on additionne goutte à goutte 1 équivalent de chloroformiate d'éthyle, soit 1,05 g. On laisse revenir à température ambiante (25°C) et on agite 2 heures.

On ajoute 10 ml d'eau au milieu réactionnel. La phase organique est extraite par :

- 10 ml d'acide chlorhydrique 1%
- 10 ml d'une solution de carbonate de sodium 1%
- 2 x 10 ml d'eau

puis séchée sur sulfate de sodium.

Après filtration sur papier, la solution est évaporée. On obtient un précipité blanc.

Masse obtenue = 2 g.

Ce précipité est recristallisé dans 45 ml d'eau.

Masse obtenue = 1,60 g
Rendement = 60%
P.F. = 134°C

Analyse élémentaire pour $C_{13}H_{20}N_4O_3$ ; PM = 280

| | C | H | N | O |
|---|---|---|---|---|
| Calculé | 55,71 | 7,14 | 20,00 | 17,14 |
| Trouvé | 55,80 | 7,18 | 20,09 | 17,05 |

Les spectres de RMN $^{13}C$ et de masse sont conformes à la structure.

EXEMPLE 12

Acétamido-2 méthyl-4 diméthylamino-6 pyrimidine oxyde-3

Suivant le mode opératoire décrit dans l'exemple 11, on fait réagir le chlorure d'acétyle, en présence de triéthylamine, sur l'amino-2 méthyl-4 diméthylamino-6 pyrimidine oxyde-3.

Température : 25°C
Temps : 2 heures
Recristallisation dans l'acétate d'éthyle-méthanol (50/50)
Rendement = 10%
P.F. = 208°C

Analyse élémentaire pour $C_9H_{14}N_4O_2$ ; PM = 210

| | C | H | N | O |
|---|---|---|---|---|
| Calculé | 51,43 | 6,67 | 26,67 | 15,24 |
| Trouvé | 51,32 | 6,66 | 26,66 | 15,36 |

Les spectres de RMN $^{13}C$ et de masse sont conformes à la structure.

EXEMPLE 13

Méthyl-7 pyrrolidino-5-2H-oxadiazolo-1,2,4 [2,3a] pyrimidine-oxo-2

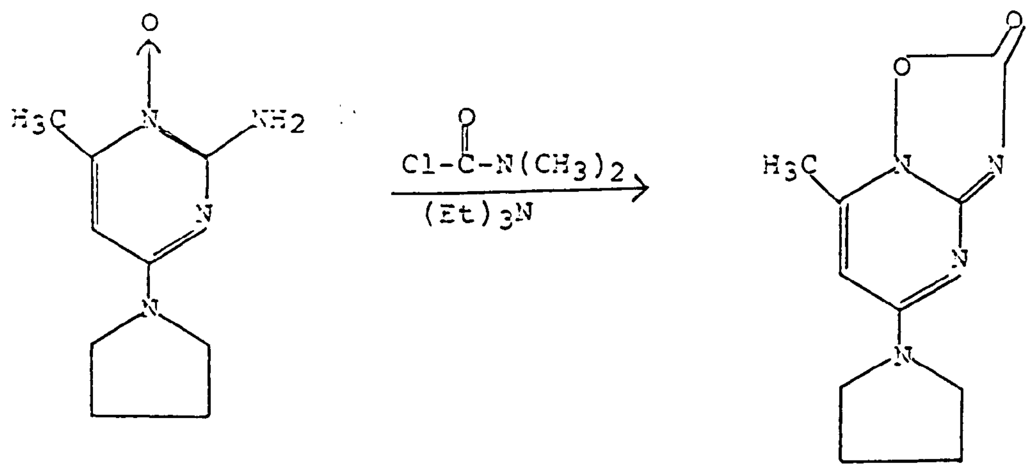


Mode opératoire :

Dans un tricol de 50 ml muni d'un agitateur magnétique, d'un thermomètre et d'une ampoule à addition, on place 2 g (1,25.$10^{-2}$mole) d'amino-2 méthyl-4 pyrrolidino-6 pyrimidine oxyde-3 en suspension dans 20 ml de dichlorométhane préalablement séché sur tamis moléculaire. On ajoute 2 équivalents de triéthylamine, soit 2,9 ml. On refroidit le milieu réactionnel à 0°C. On additionne goutte à goutte 2 équivalents de diméthylcarbamoylchlorure, soit 2,2 g. On laisse revenir à température ambiante (25°C) et on agite 2 heures.

On refroidit à 0°C puis on filtre le précipité correspondant au chlorhydrate de triéthylamine. La solution est évaporée à sec. On reprend le solide obtenu dans de l'éthanol. Après avoir agité 1 heure, on filtre le précipité. Ce précipité est recristallisé dans de l'éthanol.

Masse obtenue = 0,5 g
Rendement = 23%
P.F. = 255°C (décomposition)

Analyse élémentaire pour $C_{10}H_{12}N_4O_2.1/5H_2O$ ; PM = 220

| | C | H | N | O |
|---|---|---|---|---|
| Calculé | 53,66 | 5,54 | 25,05 | 15,74 |
| Trouvé | 53,96 | 5,51 | 25,25 | 15,66 |

Les spectres de RMN $^1$H et de masse sont conformes à la structure attendue.

EXEMPLE 14

Amino-2 méthyl-4 méthoxy-6 pyrimidine oxyde-3

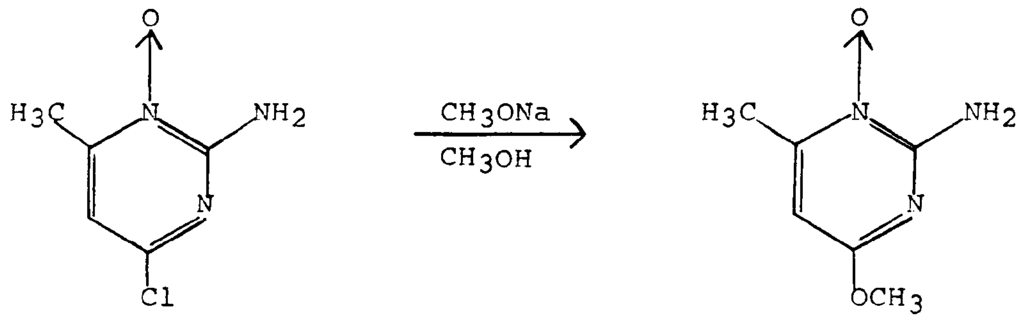


On dissout 10 g de sodium dans 750 ml de méthanol. On ajoute 50 g d'amino-2 méthyl-4 chloro-6 pyrimidine oxyde-3 et on porte le milieu réactionnel à reflux pendant 4 heures. On évapore le solvant, le mélange est dissous dans 250 ml d'eau et 30 ml d'acide chlorhydrique concentré, filtré puis basifié par addition de soude. Le précipité blanc obtenu est recristallisé dans de l'eau.

Rendement = 43%

P.F. = 210°C

Analyse élémentaire pour $C_6H_9N_3O_2.0,15\ H_2O$ ; PM = 155

| | C | H | N | O |
|---|---|---|---|---|
| Calculé | 45,66 | 5,90 | 26,63 | 21,81 |
| Trouvé | 45,77 | 5,93 | 26,56 | 21,84 |

Les spectres $^{13}C$ RMN et de masse sont conformes à la structure attendue.

EXEMPLE 15

Amino-2 méthyl-4 éthoxy-6 pyrimidine oxyde-3

On suit le mode opératoire décrit à l'exemple 14, en utilisant l'éthanol.

Température : 80°C

Temps : 4 heures

Après mise à sec du mélange réactionnel et addition d'éther, on filtre le précipité obtenu. On évapore les eaux-mères et on précipite par de l'acétone. Le précipité blanc obtenu est recristallisé dans un mélange acétone/eau.

Rendement = 25%

P.F. = 155°C

Analyse élémentaire pour $C_7H_{11}N_3O_2$ ; PM = 169

| | C | H | N | O |
|---|---|---|---|---|
| Calculé | 49,70 | 6,51 | 24,85 | 18,93 |
| Trouvé | 49,34 | 6,6 | 24,63 | 18,69 |

Les spectres $^1H$, $^{13}C$ RMN et de masse sont conformes à la structure attendue.

EXEMPLE 16

Amino-2 méthyl-4 butyloxy-6 pyrimidine oxyde-3

On suit le mode opératoire décrit à l'exemple 14, en utilisant le butanol.
Température : 118°C
Temps : 4 heures
Recristallisation dans un mélange éther de Pétrole/Acétone.
Rendement = 34%
P.F. = 137°C

Analyse élémentaire pour $C_9H_{15}N_3O_2$ ; PM = 197

| | C | H | N | O |
|---|---|---|---|---|
| Calculé | 54,82 | 7,61 | 21,32 | 16,24 |
| Trouvé | 54,74 | 7,65 | 21,20 | 16,41 |

Les spectres $^1H$ RMN et de masse sont conformes à la structure attendue.

EXEMPLE 17

Amino-2 méthyl-4(méthyl-1)éthyloxy-6 pyrimidine oxyde-3

On suit le mode opératoire décrit à l'exemple 14, mais en utilisant l'isopropanol.
Température : reflux
Temps : 48 heures
Recristallisation dans un mélange acétonitrile-eau (98/2)
Rendement = 10%
P.F. = 190°C

Analyse élémentaire pour $C_8H_{13}N_3O_2$ ; PM = 183

| | C | H | N | O |
|---|---|---|---|---|
| Calculé | 52,46 | 7,10 | 22,95 | 17,49 |
| Trouvé | 52,44 | 7,20 | 22,92 | 17,58 |

Les spectres $^1H$ RMN et de masse sont conformes à la structure attendue.

EXEMPLE 18

Amino-2 méthyl-4(diméthyl-2,2)propyloxy-6 pyrimidine oxyde-3

On dissout 0,32 g de sodium dans 50 ml de diméthyl-2,2 propanol-1 à chaud. On ajoute 2 g (0,0125 mol) d'amino-2 méthyl-4 chloro-6 pyrimidine oxyde-3 et on porte le milieu réactionnel à 105°C pendant 22 heures. On verse le mélange encore chaud dans 300 ml d'une solution aqueuse de soude à 10%. On extrait avec de l'acétate d'éthyle et on sèche la phase organique avec du sulfate de sodium. On concentre et on obtient une huile brune. On filtre sur gel de silice en utilisant un gradient d'élution avec acétate d'éthyle et méthanol. Après évaporation, on obtient un produit brut solide qui est recristallisé dans un mélange acétonitrile-eau (98/2).
Rendement = 11%
P.F. = 213°C

Analyse élémentaire pour $C_{10}H_{17}N_3O_2$ ; PM = 211

| | C | H | N | O |
|---|---|---|---|---|
| Calculé | 56,87 | 8,06 | 19,90 | 15,16 |
| Trouvé | 56,74 | 8,04 | 19,80 | 15,15 |

Les spectres $^1H$ RMN et de masse sont conformes à la structure attendue.

EXEMPLE 19

Amino-2 méthyl-4(cyclohexyl-1)méthyloxy-6 pyrimidine oxyde-3

On suit le mode opératoire décrit à l'exemple 14, en utilisant le cyclohexylméthanol.

Température : 85°C
Temps : 5 heures
Recristallisation dans un mélange acétonitrile-eau (98/2)
Rendement = 30%
P.F. = 161°C

Analyse élémentaire pour $C_{12}H_{19}N_3O_2$, 0,1 $H_2O$ ; PM = 237

| | C | H | N | O |
|---|---|---|---|---|
| Calculé | 60,25 | 8,04 | 17,57 | 14,13 |
| Trouvé | 60,26 | 8,05 | 17,60 | 14,10 |

Les spectres $^1H$ RMN et de masse sont conformes à la structure attendue.

EXEMPLE 20

Amino-2 méthyl-4(hexenyl-5)oxy-6 pyrimidine oxyde-3

On suit le mode opératoire décrit à l'exemple 14, en utilisant l'hexène-5 ol-1.

Température : 85°C
Temps : 3 heures 15 minutes
Recristallisation dans l'acétonitrile
Rendement = 27%
P.F. = 103°C

Analyse élémentaire pour $C_{11}H_{17}N_3O_2$, 0,1 $H_2O$ ; PM = 223

| | C | H | N | O |
|---|---|---|---|---|
| Calculé | 58,66 | 7,65 | 18,66 | 15,01 |
| Trouvé | 58,68 | 7,65 | 18,52 | 14,93 |

Les spectres $^1H$ RMN et de masse sont conformes à la structure attendue.

EXEMPLE 21

Amino-2 méthyl-4 benzyloxy-6 pyrimidine oxyde-3

On suit le mode opératoire décrit à l'exemple 14, en utilisant l'alcool benzylique.

Température : 83°C

Temps : 2 heures 30 minutes

Recristallisation dans un mélange acétonitrile-eau (98/2)

Rendement = 45%

P.F. = 188°C

Analyse élémentaire pour $C_{12}H_{13}N_3O_2$ ; PM = 231

| | C | H | N | O |
|---|---|---|---|---|
| Calculé | 62,33 | 5,62 | 18,18 | 13,85 |
| Trouvé | 62,40 | 5,59 | 18,06 | 13,89 |

Les spectres $^1H$ RMN et de masse sont conformes à la structure attendue.

EXEMPLE 22

Amino-2 méthyl-4(diméthyl-2,4)phényloxy-6 pyrimidine oxyde-3

On suit le mode opératoire décrit à l'exemple 14, en utilisant le diméthyl-2,4 phénol.

Température : 75°c

Temps : 4 heures

Recristallisation dans un mélange acétonitrile-eau (95/5)

Rendement = 60%

P.F. = 214°C

Analyse élémentaire pour $C_{13}H_{15}N_3O_2$ ; PM = 245

| | C | H | N | O |
|---|---|---|---|---|
| Calculé | 63,67 | 6,12 | 17,14 | 13,06 |
| Trouvé | 63,60 | 6,16 | 17,16 | 13,19 |

Les spectres $^{13}C$, $^1H$ RMN et de masse sont conformes à la structure attendue.

EXEMPLE 23

Amino-2 méthyl-4 méthylthio-6 pyrimidine oxyde-3

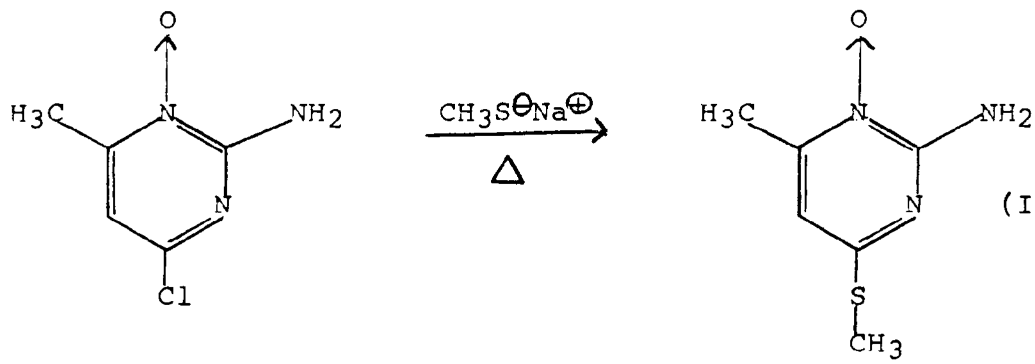


(I)

Le composé (I) est obtenu par action du méthylthiolate de sodium, à chaud, sur l'amino-2 méthyl-4 chloro-6 pyrimidine oxyde-3.

Mode opératoire :

5 g ($3,12.10^{-2}$mole) d'amino-2 méthyl-4 chloro-6 pyrimidine oxyde-3 sont introduits dans 75 ml de diméthoxy-1,2 éthane; on ajoute 5,46 g ($7,81.10^{-2}$mole) de méthylthiolate de sodium puis on porte à reflux jusqu'à disparition du substrat de départ ($t_{reaction}$=55 heures). Le précipité obtenu, après filtration du milieu réactionnel, est dissous dans le méthanol et additionné d'éthanol chlorhydrique jusqu'à pH=2; le chlorhydrate du composé (I) est isolé, puis la base associée est libérée par ajout d'une solution de méthylate de sodium à 30% dans le méthanol; après avoir éliminé les sels par précipitation à l'éther éthylique, le filtrat résultant est évaporé à sec puis recristallisé dans le système acétonitrile/éthanol (70/30). On obtient 1,55 g du composé (I).

Rendement = 29%
P.F. = 172°C

Analyse élémentaire pour $C_6H_9N_3OS$ ; PM = 171

| | C | H | N | O | S |
|---|---|---|---|---|---|
| Calculé | 42,10 | 5,26 | 24,56 | 9,35 | 18,71 |
| Trouvé | 42,17 | 5,31 | 24,65 | 9,45 | 18,47 |

Les spectres $^{13}C$ RMN et de masse sont conformes à la structure attendue.

EXEMPLE 24

Amino-2 méthyl-4 éthylthio-6 pyrimidine oxyde-3

5 g ($3,12.10^{-2}$mole) d'amino-2 méthyl-4 chloro-6 pyrimidine oxyde-3 sont introduits en suspension dans 75 ml de diméthoxy-1,2 éthane; on ajoute 3,92 g ($4,68.10^{-2}$mole) d'éthylthiolate de sodium, puis on porte à reflux jusqu'à disparition du substrat de départ ($t_{reaction}$=18 heures). Le précipité issu du milieu réactionnel est agité en suspension dans 30 ml d'eau puis filtré, séché et recristallisé dans l'acétonitrile. On obtient 2,1 g du composé attendu.

Rendement = 36%
P.F. = 152°C

Analyse élémentaire pour $C_7H_{11}N_3OS$ ; PM = 185

| | C | H | N | O | S |
|---|---|---|---|---|---|
| Calculé | 45,40 | 5,94 | 22,70 | 8,65 | 17,29 |
| Trouvé | 45,33 | 5,95 | 22,82 | 8,78 | 17,32 |

Les spectres $^1H$ RMN et de masse sont conformes à la structure attendue.

EXEMPLE 25

Amino-2 méthyl-4 pyrimidine oxyde-3

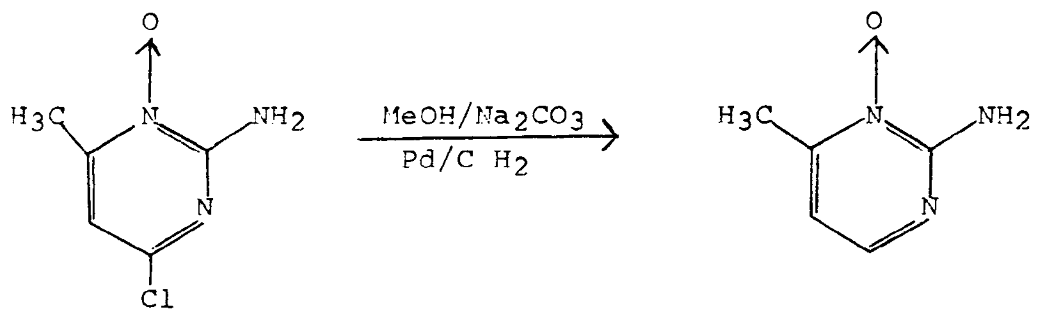


On mélange 15 g d'amino-2 méthyl-4 chloro-6 pyrimidine oxyde-3, 11 g de carbonate de sodium, 2,4 g de palladium/charbon à 10% dans 1 litre de méthanol. On hydrogénolyse dans un appareil de PARR sous $2 \times 10^5$ Pa pendant 2 heures 30 minutes. On filtre le milieu réactionnel puis on évapore le solvant. Le précipité est extrait par de l'acétate d'éthyle bouillant.

Rendement = 20%

P.F. = 145°C

Analyse élémentaire pour $C_5H_7N_3O$ ; PM = 125

| | C | H | N | O |
|---|---|---|---|---|
| Calculé | 48,00 | 5,6 | 33,6 | 12,8 |
| Trouvé | 47,97 | 5,6 | 33,7 | 12,87 |

Les spectres $^{13}C$ RMN et de masse sont conformes à la structure attendue.

EXEMPLE 26

Sel interne de l'hydroxyde de l'amino-2 méthyl-4 diméthylamino-6 sulfooxy-3 pyrimidinium

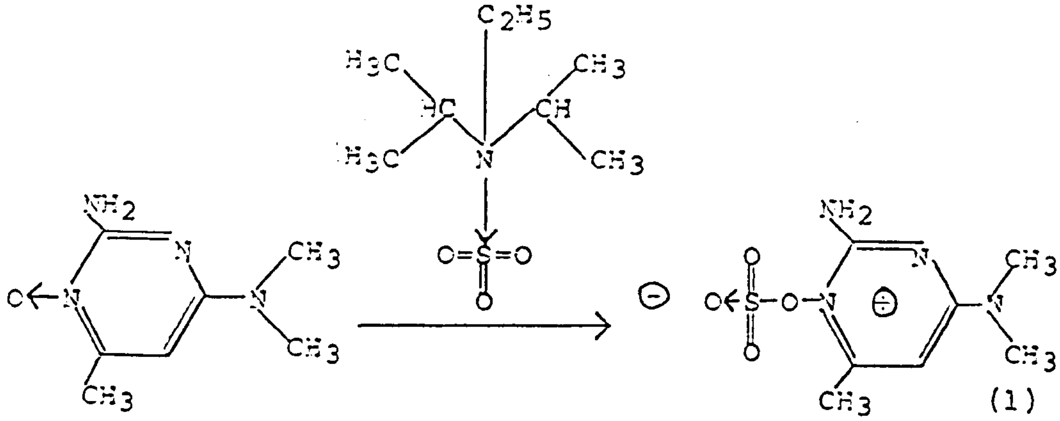

(1)

A une solution de 1,03 ml (0,006 mole) de N,N-diisopropyléthylamine dans 8 ml de chloroforme refroidie dans la glace, on ajoute, sous agitation, 0,2 ml (0,003 mole) d'acide chlorosulfonique. Après attente de 30 minutes, on ajoute 0,252 g (0,0015 mole) d'amino-2 méthyl-4 diméthylamino-6 pyrimidine oxyde-3 et on maintient 2 heures entre 0° et 5°C, sous azote. On filtre le précipité blanc qui s'est formé, puis on le lave avec un peu de chloroforme.

Après recristallisation dans un mélange diméthylformamide-eau, on obtient 0,11 g du composé (1) qui se décompte à 250°C. Le rendement est de 30%.

Analyse élémentaire pour $C_7H_{12}N_4O_4S$ ; PM = 248

| | C | H | N | O | S |
|---|---|---|---|---|---|
| Calculé | 33,88 | 4,84 | 22,58 | 25,80 | 12,90 |
| Trouvé | 33,90 | 4,86 | 22,62 | 25,82 | 12,81 |

Spectre de masse : conforme.

EXEMPLE 27

Sel interne de l'hydroxyde de l'amino-2 méthyl-4 pyrrolidino-6 sulfooxy-3 pyrimidinium

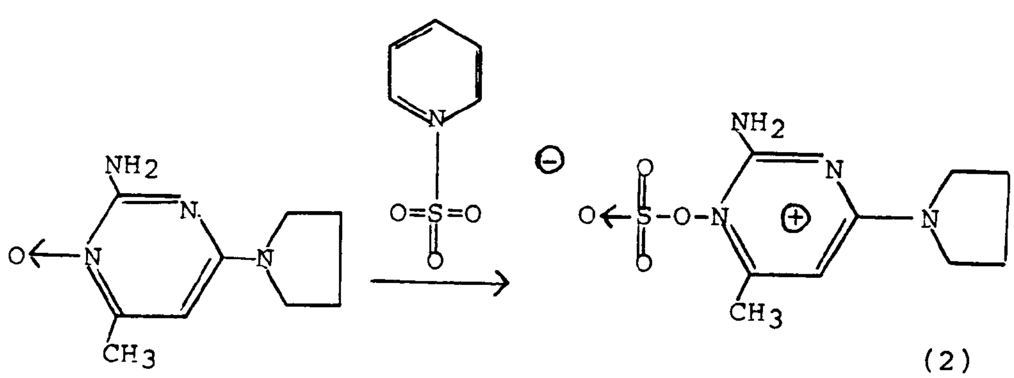

(2)

Dans une suspension de 0,194 g (0,001 mole) d'amino-2 méthyl-4 pyrrolidino-6 pyrimidine oxyde-3, on ajoute sous agitation 0,32 g (0,002 mole) de complexe de trioxyde de soufre-pyridine et on maintient à température ambiante pendant 3 heures. On dilue la solution obtenue par 15 g d'eau glacée. On filtre le précipité blanc, puis on le lave avec un peu d'eau glacée.

Après recristallisation dans un mélange diméthylformamide-eau, on obtient 0,085 g du composé (2) qui se décompose à 260°C.

Le rendement est de 31%.

Analyse élémentaire pour $C_9H_{14}N_4O_4S$ ; PM = 274

| | C | H | N | O | S |
|---|---|---|---|---|---|
| Calculé | 39,42 | 5,11 | 20,44 | 23,36 | 11,68 |
| Trouvé | 39,35 | 5,16 | 20,33 | 23,36 | 11,61 |

Spectre de masse : conforme.

EXEMPLE 28

Sel interne de l'hydroxyde de l'amino-2 méthyl-4 pipéridino-6 sulfooxy-3 pyrimidinium

On suit le mode opératoire décrit à l'exemple 27, en utilisant l'amino-2 méthyl-4 pipéridino-6 pyrimidine oxyde-3.

Le produit obtenu recristallisé dans un mélange diméthylformamide-eau est un monohydrate qui se décompose à 184°C.

Le rendement est de 73%.

Analyse élémentaire pour $C_{10}H_{18}N_4O_5S$ ; PM = 306

| | C | H | N | O | S |
|---|---|---|---|---|---|
| Calculé | 39,21 | 5,88 | 18,30 | 26,14 | 10,46 |
| Trouvé | 39,27 | 6,08 | 18,48 | 26,02 | 10,44 |

Spectre de masse : conforme.

Spectre $^1H$ RMN : conforme.

EXEMPLE 29

Sel interne de l'hydroxyde de l'amino-2 méthyl-4 morpholino-6 sulfooxy-3 pyrimidinium

On suit le mode opératoire décrit à l'exemple 26, en utilisant l'amino-2 méthyl-4 morpholino-6 pyrimidine oxyde-3.

Le temps de réaction est de 3 heures et la température est maintenue entre 5 et 10°C.

Le produit obtenu recristallisé dans un mélange diméthylformamide-eau se décompose à 250°C.

Le rendement est de 56%.

Analyse élémentaire pour $C_9H_{14}N_4O_5S$ ; PM = 290

| | C | H | N | O | S |
|---|---|---|---|---|---|
| Calculé | 37,24 | 4,83 | 19,31 | 27,59 | 11,03 |
| Trouvé | 37,29 | 4,81 | 19,39 | 27,83 | 10,96 |

Spectre de masse : conforme.

EXEMPLE 30

Sel interne de l'hydroxyde de l'amino-2 méthyl-4 thiomorpholino-6 sulfooxy-3 pyrimidinium

On suit le mode opératoire décrit à l'exemple 26, en utilisant l'amino-2 méthyl-4 thiomorpholino-6 pyrimidine oxyde-3.

Le temps de réaction est de 1 heure 30 minutes.

Le produit obtenu recristallisé dans un mélange diméthylformamide-eau se décompose au-dessus de 260°C.

Le rendement est de 74%.

Analyse élémentaire pour $C_9H_{14}N_4O_4S_2$ ; PM = 306

| | C | H | N | O | S |
|---|---|---|---|---|---|
| Calculé | 35,29 | 4,57 | 18,30 | 20,91 | 20,91 |
| Trouvé | 35,15 | 4,50 | 18,35 | 21,10 | 20,88 |

Spectre de masse : conforme.

EXEMPLE 31

Sel interne de l'hydroxyde de l'amino-2 méthyl-4 (méthyl-4 pipérazino)-6 sulfooxy-3 pyrimidinium

On suit le mode opératoire décrit à l'exemple 26, en utilisant l'amino-2 méthyl-4 (méthyl-4 pipérazino)-6 pyrimidine oxyde-3.

Le temps de réaction est de 3 heures.

Le produit obtenu recristallisé dans un mélange diméthylformamide-eau se décompose à 265°C.

Le rendement est de 20%.

Analyse élémentaire pour $C_{10}H_{17}N_5O_4S$, 0,15 $H_2O$ ; PM = 303

| | C | H | N | O | S |
|---|---|---|---|---|---|
| Calculé | 39,27 | 5,65 | 22,91 | 21,68 | 10,47 |
| Trouvé | 39,18 | 5,72 | 23,01 | 21,68 | 10,46 |

Spectre de masse : conforme.

EXEMPLE 32

Sel interne de l'hydroxyde de l'amino-2 méthyl-4 méthoxy-6 sulfooxy-3 pyrimidinium

On suit le mode opératoire décrit à l'exemple 26, en utilisant l'amino-2 méthyl-4 méthoxy-6 pyrimidine oxyde-3.

Le temps de réaction est de 1 heure 30 minutes.

Le rendement en produit brut est de 60%.

Spectre de masse : conforme.

EXEMPLE 33

Sel interne de l'hydroxyde de l'amino-2 méthyl-4 éthoxy-6 sulfooxy-3 pyrimidinium

A une solution de 1,03 ml (0,006 mole) de N,N-diisopropyléthylamine dans 8 ml de chloroforme refroidie dans la glace, on ajoute, sous agitation, 0,2 ml (0,003 mole) d'acide chlorosulfonique. Après une attente de 30 minutes, on ajoute 0,253 g (0,0015 mole) d'amino-2 méthyl-4 éthoxy-6 pyrimidine oxyde-3 et on maintient 2 heures entre 0°C et 5°C, sous azote. Le solvant est évaporé. Le résidu repris dans un peu d'eau laisse cristalliser un produit blanc que l'on filtre et sèche.

Le rendement en produit brut est de 25%.

Spectre de masse : conforme.

EXEMPLE 34

Amino-2 tétraméthylène-4,5 pipéridino-6 pyrimidine oxyde-3

1ère partie:

Préparation de l'amino-2 tétraméthylène-4,5 hydroxy-6 pyrimidine.

Dans un tricol, muni d'un réfrigérant, d'un thermomètre et d'une entrée d'argon, on dissout 2,15g (9,35 $10^{-2}$m) de sodium dans 70 ml d'éthanol absolu puis on ajoute 8,95g (9,37 $10^{-2}$m) de chlorure de guanidinium par portions solides en maintenant la température à 25°C.

On procède ensuite à une coulée progressive de 16,66ml d'éthylcyclohexanone-2 carboxylate à 90% (9,37 $10^{-2}$m) introduire au préalable dans une ampoule à brome; le temps d'écoulement est de 20 minutes et la température de la réaction exotherme se développe jusqu'à 45°C.

Lorsque la température du milieu réactionnel est devenue à l'ambiante, on filtre le précipité, on le lave abondamment à l'eau pour éliminer le chlorure de sodium, puis à l'éthanol; la masse brute ainsi obtenue est recristallisée dans l'eau.

Masse recristallisée : 11,84g

Rendement = 76,3%

Analyses :

P.F. 300°C

Analyse élémentaire pour $C_8H_{11}N_3O;0,1H_2O$ ; PM = 165

| | C | H | N | O |
|---|---|---|---|---|
| Calculé | 57,55 | 6,65 | 25,18 | 10,55 |
| Trouvé | 57,57 | 6,73 | 25,09 | 10,56 |

Les spectres RMN $^1$H et de masse sont conformes à la structure attendue.

2ème partie :

Préparation de l'amino-2 tétraméthylène-4,5 chloro-6 pyrimidine.

Dans un tricol de 250ml, muni d'un thermomètre, d'un réfrigérant surmonté d'une garde à chlorure de calcium et d'une agitation magnétique, on introduit 10g (6 $10^{-2}$m) d'amino-2 tétraméthylène-4,5 hydroxy-6 pyrimidine dans 100 ml d'oxychlorure de phosphore.

On chauffe le milieu à 100°C pendant 1/2 heure, puis on laisse revenir à température ambiante. L'oxychlorure de phosphore est évaporé; l'huile obtenue est versée lentement sur une solution aqueuse d'ammoniaque à 6,6%(300ml) en maintenant la température inférieure à 10°C.

On agite 1/2 heure et on filtre le précipité blanc obtenu qu'on lave à l'eau jusqu'à neutralité.

La masse brute ainsi récupérée est recristallisée dans l'éthanol.

Masse recristallisée = 4g

Rendement = 36,4%

Analyses :

P.F. = 207°C

Analyse élémentaire pour $C_8H_{10}N_3Cl$ ; PM = 183,5

| | C | H | N | Cl |
|---|---|---|---|---|
| Calculé | 52,32 | 5,45 | 22,89 | 19,34 |
| Trouvé | 52,27 | 5,49 | 22,98 | 19,46 |

Les spectres de RMN $^1H$ et de masse sont conformes à la structure attendue.

3ème partie :

Préparation de l'amino-2 tétraméthylène-4,5 chloro-6 pyrimidine oxyde-3

Dans un tricol d'un litre muni d'un thermomètre et d'une agitation magnétique, on introduit 30g ($16,34.10^{-2}$m) d'amino-2 tétraméthylène-4,5 chloro-6 pyrimidine en suspension dans 600 ml de méthanol; on ajoute alors 76,2g (1,5 équivalent) d'acide métachloroperbenzoïque à 55% en maintenant la température à 5°C.

On laisse ensuite agiter 4 heures à l'ambiante (25°C).

Le milieu réactionnel est filtré et le précipité isolé est lavé au méthanol froid, puis à l'éther éthylique.

Masse obtenue = 28,5 g

Rendement = 87%

Analyses :

P.F. = 188°C

Analyse élémentaire pour $C_8H_{10}ClN_3O$ ; PM = 199,5

| | C | H | N | O | Cl |
|---|---|---|---|---|---|
| Calculé | 42,12 | 5,01 | 21,05 | 8,02 | 17,79 |
| Trouvé | 48,07 | 5,05 | 21,09 | 7,95 | 17,85 |

Les spectres de RMN $^1H$ et de masse sont conformes à la structure attendue.

4ème partie :

Préparation de l'amino-2 tétraméthylène-4,5 pipéridino-6 pyrimidine oxyde-3

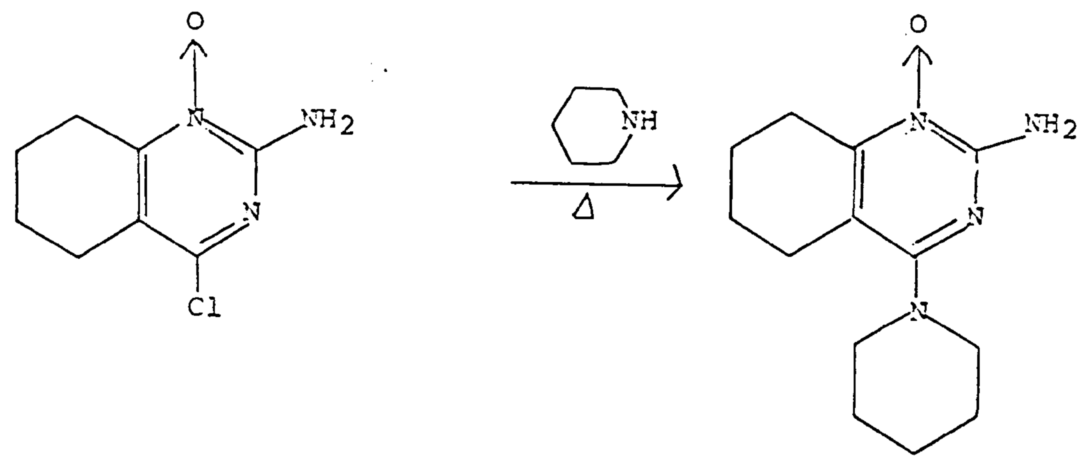


Mode opératoire :

10 g ($5.10^{-2}$m) d'amino-2 tétraméthylène-4,5 chloro-6 pyrimidine oxyde-3 sont introduits dans 100 ml de pipéridine; le milieu réactionnel est chauffé à reflux pendant 8 heures, refroidi à l'ambiante, puis filtré sur fritté. Le précipité obtenu est additionné par portions solides à une solution réfrigérée de 30 $cm^3$ d'eau dans laquelle on a dissous 2g de soude et 3g de chlorure de sodium. On agite 1 heure, on filtre, puis on lave à l'eau jusqu'à neutralité ; les cristaux blancs sont recristallisés dans un mélange eau/éthanol (50/50).

Masse obtenue = 5,4 g

Rendement = 43,5%

Analyses :

P.F. = 178°C

Analyse élémentaire pour : $C_{13}H_{20}N_4O;0,3H_2O$ PM=248

| | C | H | N | O |
|---|---|---|---|---|
| Calculé | 61,56 | 5,13 | 22,09 | 8,21 |
| Trouvé | 62,02 | 8,11 | 22,19 | 8,24 |

Les spectres de RMN $^1H$ et de masse sont conformes à la structure attendue.

EXEMPLE 35

Préparation d'amino-2 tétraméthylène-4,5 butoxy-6 pyrimidine oxyde-3

Mode opératoire :

Dans un tricol, muni d'un réfrigérant, d'un thermomètre et d'une entrée d'argon, on ajoute 1,72g (7,5 $10^{-2}$m) de sodium dans 100 ml de butanol anhydre, on agite pendant 3/4 d'heure en laissant se développer l'exothermicité liée à la formation du butylate de sodium. On additionne ensuite par portions solides, 10g ($5.10^{-2}$m) d'amino-2 tétraméthylène-4,5 chloro-6 pyrimidine oxyde-3 puis on chauffe au reflux du butanol durant 1heure.

Après refroidissement du milieu réactionnel, on filtre et on ajuste le filtrat à neutralité par addition d'éthanol chlorhydrique; le chlorure de sodium est éliminé , puis la phase organique est lavée avec 30ml d'eau distillée, évaporée à sec et le résidu huileux résultant est précipité par addition d'éther éthylique. On agite dans un bain

d'eau glacée durant 1/2 heure, puis on filtre .

La masse brute obtenue (9g) est recristallisée dans un mélange acéonitrile/éthanol (50/50), on récupère ainsi 4,65g du composé pur.

Rendement = 39%

Analyses :

P.F. = 166°C

Analyse élémentaire pour : $C_{12}H_{19}N_3O_2$ PM=237

| | C | H | N | O |
|---|---|---|---|---|
| Calculé | 60,76 | 8,02 | 17,72 | 13,50 |
| Trouvé | 60,76 | 8,06 | 17,78 | 13,69 |

Les spectres de RMN $^{13}C$ et de masse sont conformes à la structure attendue.

EXEMPLE 36

Préparation d'amino-2 méthyl-4 anilino-6 pyrimidine oxyde-3

Dans un tricol, muni d'un réfrigérant, d'un thermomètre et d'une agitation magnétique, on place 20g d'amino-2 méthyl-4 chloro-6 pyrimidine oxyde-3, 25,65g d'aniline et 100ml d'éthanol. Le milieu réactionnel est porté à 55°C pendant 4 heures.

Après être revenu à température ambiante, on filtre sur papier, puis on évapore à sec, l'huile est reprise dans 10 ml d'éthanol, puis amenée à pH = 4 par addition d'éthanol chlorhydrique. On additionne 100 ml d'éther éthylique. Un produit beige précipite. Ce précipité est filtré, lavé par de l'éther éthylique, séché sous vide, puis repris dans 50 ml de soude. Après 1 heure d'agitation, le précipité est filtré, lavé par de l'eau jusqu'à neutralité, séché sous vide sur pentoxyde de phosphore. On recristallise dans 120 ml de diméthyl formamide et on obtient 3,1g d'amino-2 méthyl-4 anilino-6 pyrimidine oxyde-3.

Rendement = 11,5%

Analyses :

P.F. = 260°C

Analyse élémentaire pour : $C_{11}H_{12}N_2O$ ; PM = 216

| | C | H | N | O |
|---|---|---|---|---|
| Calculé | 61,12 | 5,50 | 25,92 | 7,40 |
| Trouvé | 61,12 | 5,50 | 25,82 | 7,62 |

Les spectres de masse et RMN $^1H$ sont conformes à la structure attendue.

EXEMPLE 37

Préparation d'amino-2 méthyl-4 (N-méthylphénéthylamino)-6 pyrimidine oxyde-3

On suit le mode opératoire décrit à l'exemple 36, en utilisant la N-méthylphénéthylamine.
Température = 40°C
Temps : 3 heures
Recristallisation dans acétonitrile
Rendement = 33%
P.F. = 170°C

Analyse élémentair pour $C_{14}H_{18}N_4O$ ; PM = 258

| | C | H | N | O |
|---|---|---|---|---|
| Calculé | 65,12 | 6,98 | 21,70 | 6,20 |
| Trouvé | 64,82 | 6,96 | 21,50 | 6,64 |

Les spectres de masse et RMN $^1H$ sont conformes à la structure attendue.

EXEMPLE 38

Préparation d'amino-2 méthyl-4 héxaméthylèneimino-6 pyrimidine oxyde-3

On suit le mode opératoire décrit à l'exemple 3, en utilisant l'hexaméthylèneimine.
Température = 80°C
Temps : 3 heures
Recristallisation dans acétonitrile/Ethanol (70/30)
Rendement = 17%
P.F. = 218°C

Analyse élémentaire pour $C_{11}H_{18}N_4O$ ; PM = 222

| | C | H | N | O |
|---|---|---|---|---|
| Calculé | 59,46 | 8,10 | 25,22 | 7,20 |
| Trouvé | 59,47 | 8,21 | 25,40 | 7,30 |

Les spectres de masse et RMN $^1H$ sont conformes à la structure attendue.

EXEMPLE 39

Préparation du sel interne de l'hydroxyde de l'amino-2 tétraméthylène-4,5 butoxy-6 sulfooxy-3 pyrimidinium

A une solution de 1,2 ml (0,007m) de N,N-diisopropyléthylamine dans 6ml de chloroforme refroidie dans la glace, est ajouté, sous agitation, 0,2 ml (0,003 m) d'acide chlorosulfonique. Après attente de 30 minutes, on ajoute 0,237g (0,001 m) d'amino-2 tétraméthylène-4,5 butoxy-6 pyrimidine oxyde-3 et on maintient 2 heures entre 0° et 5°C, sous azote. Le solvant est évaporé. Le résidu est repris dans un peu d'eau, on obtient un produit cristallin blanc que l'on filtre. Après recristallisation dans un mélange de diméthylformamide/eau, on obtient 0,13 g de cristaux blancs qui se décomposent à 122°C.
Rendement = 41%

Analyse élémentaire pour $C_{12}H_{19}N_3O_5S$;0,21 $H_2O$ PM = 317

| | C | H | N | O | S |
|---|---|---|---|---|---|
| Calculé | 44,89 | 6,05 | 13,09 | 25,9 | 9,97 |
| Trouvé | 44,89 | 6,08 | 13,09 | 26,02 | 9,93 |

Les spectres de masse et RMN $^1H$ sont conformes à la structure attendue.

EXEMPLE 40

Préparation du sel interne de l'hydroxyde de l'amino-2 méthyl-4 N-méthylphénéthylamino-6 sulfooxy-3 pyrimidinium

1ère méthode

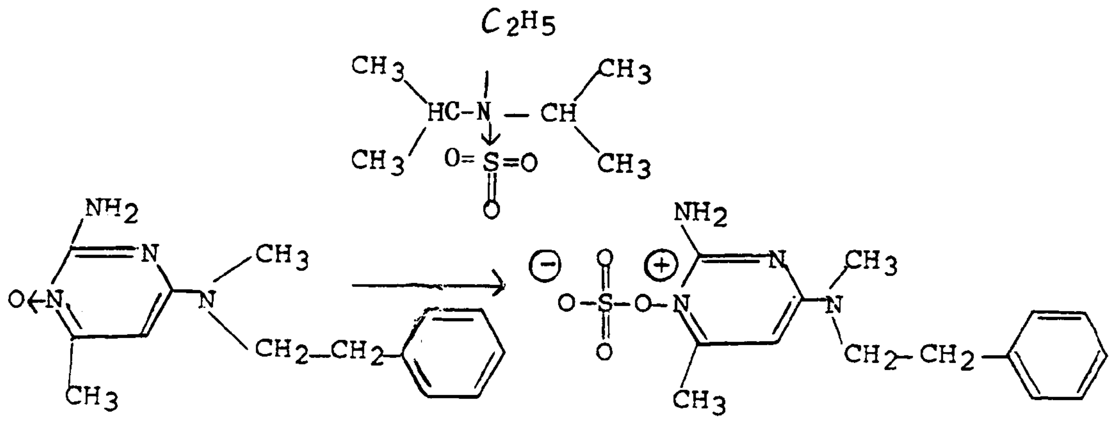

A une solution de 0,72ml (0,0042m) de N,N-diisopropyléthylamine dans 6ml de chloroforme refroidie dans la glace, est ajouté, sous agitation, 0,133 ml (0,002 m) d'acide chlorosulfonique. Après attente de 30 minutes, on ajoute 0,258g (0,001 m) d'amino-2 méthyl-4 N-méthylphénéthylamino-6 pyrimidine oxyde-3 et l'on maintient pendant 3 heures entre 0° et 5°C, sous azote. Le solvant est évaporé. On reprend le résidu dans un peu d'eau, on obtient un produit cristallin blanc que l'on filtre. Après recristallisation dans un mélange acétonitrile/eau, on obtient 0,27 g de sel interne qui se décompose à 213°C.

Rendement = 80%

Analyse élémentaire pour $C_{14}H_{18}N_4O_4S$;0,25 $H_2O$ PM = 338

| | C | H | N | O | S |
|---|---|---|---|---|---|
| Calculé | 49,05 | 5,40 | 16,35 | 19,85 | 9,34 |
| Trouvé | 48,98 | 5,29 | 16,27 | 19,94 | 9,43 |

Les spectres de masse et RMN $^1H$ sont conformes à la structure attendue.

2ème méthode

Dans une suspension de 0,258g (0,001m) d'amino-2 méthyl-4 N-méthylphénéthylamino-6 pyrimidine oxyde-3 dans 3ml de diméthyl formamide est ajouté sous agitation 0,32g (0,002m) de complexe de trioxyde de

soufre/pyridine. Après 2 heures à 15-20°C, on ajoute 0,16g (0,001m) du complexe et laisse encore 1 heure à 15-20°C. On dilue la solution obtenue par 15g d'eau glacée. On filtre le précipité blanc obtenu, et on le lave à l'eau glacée. Après recristallisation dans un mélange acétonitrile/eau, on obtient 0,24g de sulfate interne qui se décompose à 213°C.

Rendement = 71%

EXEMPLE 41

Préparation du sel interne de l'hydroxyde de l'amino-2 méthyl-4 anilino-6 sulfooxy-3 pyrimidinium

On suit le mode opératoire de la première méthode décrit à l'exemple 40 en utilisant 0,216g d'amino-2 méthyl-4 anilino-6 pyrimidine oxyde-3. Le produit obtenu recristallisé dans un mélange acétonitrile/eau, se décompose au dessus de 260°C

Rendement = 64%

Analyse élémentaire pour $C_{11}H_{12}N_4O_4S$ PM = 296

| | C | H | N | O | S |
|---|---|---|---|---|---|
| Calculé | 44,59 | 4,05 | 18,92 | 21,62 | 10,82 |
| Trouvé | 44,68 | 4,07 | 18,95 | 21,54 | 10,69 |

Les spectres de masse et RMN $^1H$ sont conformes à la structure attendue

EXEMPLE 42

Préparation du sel interne de l'hydroxyde de l'amino-2 méthyl-4 hexaméthylèneimino-6 sulfooxy-3 pyrimidinium

On suit le mode opératoire de la première méthode décrit à l'exemple 40 en utilisant 0,222g d'amino-2 méthyl-4 hexaméthylèneimine-6 pyrimidine oxyde-3. Le produit obtenu recristallisé dans un mélange acétonitrile/eau, se décompose au dessus de 195°C.

Rendement = 46%

Analyse élémentaire pour $C_{11}H_{18}N_4O_4S$ PM = 302

| | C | H | N | O | S |
|---|---|---|---|---|---|
| Calculé | 43,71 | 5,96 | 18,54 | 21,19 | 10,60 |
| Trouvé | 43,79 | 6,04 | 18,71 | 21,20 | 10,40 |

Les spectres de masse et RMN $^1H$ sont conformes à la structure attendue.

EXEMPLES DE FORMULATION

On prépare une lotion destinée au traitement de la chute des cheveux, ayant la composition suivante :

Lotion 1

| | | |
|---|---|---|
| - Amino-2 méthyl-4 pipéridino-6 pyrimidine oxyde-3 (exemple 1) | | 5,0 g |
| - Propylèneglycol | | 23,0 g |
| - Ethanol | | 55,0 g |
| - Eau | qsp | 100,0 g |

Lotion 2

| | | |
|---|---|---|
| - Amino-2 méthyl-4 pipéridino-6 pyrimidine oxyde-3 (exemple 1) | | 2,0 g |
| - Ethanol | | 49,0 g |
| - Eau | qsp | 49,0 g |

Lotion 3

| | |
|---|---|
| - Amino-2 méthyl-4 morpholino-6 pyrimidine oxyde-3 (exemple 3) | 2,0 g |
| - Propylèneglycol | 4,9 g |
| - Ethanol | 93,1 g |

Lotion 4

| | | |
|---|---|---|
| - Amino-2 méthyl-4 diméthylamino-6 pyrimidine oxyde-3 (exemple 7) | | 5,0 g |
| - Propylèneglycol | | 23,0 g |
| - Ethanol | | 55,0 g |
| - Eau | qsp | 100,0 g |

Lotion 5

| | |
|---|---|
| - Amino-2 méthyl-4 diméthylamino-6 pyrimidine oxyde-3 (exemple 7) | 2,0 g |
| - Propylèneglycol | 4,9 g |
| - Ethanol | 93,1 g |

Lotion 6

| | | |
|---|---|---|
| - Amino-2 méthyl-4 butyloxy-6 pyrimidine oxyde-3 (exemple 16) | | 8,0 g |
| - Propylèneglycol | | 23,0 g |
| - Ethanol | | 55,0 g |
| - Eau | qsp | 100,0 g |

Lotion 7

| | |
|---|---|
| - Amino-2 méthyl-4 méthoxy-6 pyrimidine oxyde-3 (exemple 14) | 1,0 g |
| - Propylèneglycol | 5,0 g |
| - Ethanol | 94,0 g |

Lotion 8

| | |
|---|---|
| - Amino-2 méthyl-4 éthoxy-6 pyrimidine oxyde-3 (exemple 15) | 5,0 g |
| - Ethanol | 47,5 g |
| - Eau | 47,5 g |

Lotion 9

| | | |
|---|---|---|
| - Amino-2 méthyl-4 pyrimidine oxyde-3 (exemple 25) | | 3,0 g |
| - Ethanol | | 30,0 g |
| - Propylèneglycol | | 20,0 g |
| - Eau | qsp | 100,0 g |

Lotion 10

| | |
|---|---|
| - Amino-2 méthyl-4 pyrimidine oxyde-3 (exemple 25) | 2,0 g |
| - Propylèneglycol | 4,9 g |
| - Ethanol | 93,1 g |

On applique 1 à 2 g de chacune de ces lotions sur les zones alopéciques du cuir chevelu, à raison d'une application par jour pendant 7 jours par semaine et ceci pendant 3 mois.

## Revendications

### Revendications pour les Etats contractants suivants : AT, BE, CH, DE, DK, FR, GB, IT, LI, NL, SE

**1.** Composition destinée à être utilisée pour freiner la chute des cheveux et pour induire et stimuler leur croissance, caractérisée par le fait qu'elle contient dans un milieu cosmétiquement acceptable, au moins un composé répondant à la formule suivante :

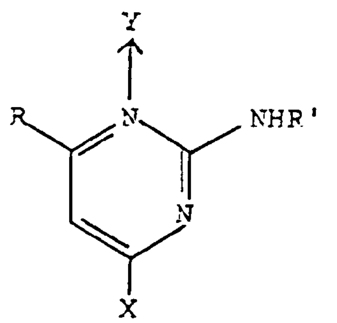

(I)

dans laquelle :

R désigne un atome d'hydrogène, un radical alkyle, saturé et linéaire en $C_1$-$C_8$ ou forme avec le cycle pyrimidine un cycle hydrocarboné de formule :

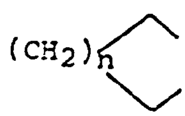

où n est égal à 1, 2 ou 3;

X désigne :

(i) un atome d'hydrogène;

(ii) un groupement

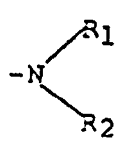

,

dans lequel

$R_1$ et $R_2$, identiques ou différents, désignent un atome d'hydrogène, un groupe alkyle saturé en $C_1$-$C_{12}$, linéaire ou ramifié, pouvant être substitué par un atome d'halogène ou un radical trifluorométhyle, un groupe alcényle linéaire en $C_2$-$C_{12}$, un groupe cycloalkyle en $C_3$-$C_{10}$, un groupe aralkyle en $C_7$-$C_{12}$ ou un groupement aryle répondant à la formule :

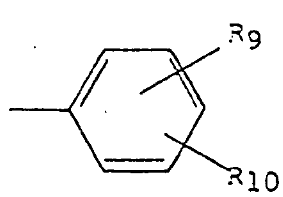

dans laquelle $R_9$ et $R_{10}$, identiques ou différents, désignent hydrogène, alkyle en $C_1$-$C_4$, hydroxyle, alcoxy en $C_1$-$C_4$ ou halogène;

$R_1$ et $R_2$ peuvent former avec l'atome d'azote auquel ils sont reliés, un hétérocycle, saturé ou insaturé, choisi parmi : aziridino, azétidino, pyrrolidino, pipéridino, hexaméthylèneimino, heptaméthylèneimino, octaméthylèneimino, tétrahydro pyridino, dihydropyridino, pyrrole, pyrazole, imidazole, triazole, alkyl-4 pipérazino, morpholino, thiomorpholino;

(iii) un groupement $-OR_3$, dans lequel :

$R_3$ désigne un radical alkyle saturé en $C_1$-$C_{12}$, linéaire ou ramifié, pouvant être substitué par un atome d'halogène ou un radical trifluorométhyle, un radical alcényle linéaire en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un atome d'halogène, un radical alkyle en $C_1$-$C_6$, un radical alcoxy en $C_1$-$C_6$, ou un radical trifluorométhyle;

(iv) un groupement $-SR_4$, dans lequel $R_4$ a la même signification que $R_3$;

Y désigne un atome d'oxygène ou un groupement $-OSO_3^{\ominus}$;

R' désigne un atome d'hydrogène ou l'un des radicaux suivants :

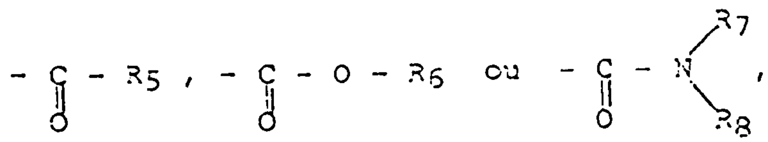

dans lesquels :

$R_5$ et $R_6$ représentent des radicaux alkyle inférieurs en $C_1$-$C_4$;

$R_7$ et $R_8$ désignent un atome d'hydrogène ou un radical alkyle inférieur en $C_1$-$C_4$, à condition qu'ils ne désignent pas simultanément un atome d'hydrogène;

ou ses sels d'addition d'acides physiologiquement acceptables, sous réserve que lorsque R' désigne hydrogène, X ne désigne pas $-NR_1R_2$ et que le composé de formule (I) soit différent de amino-2 pyrimidine oxyde-3 ou (éthoxycarbonylamino)-2 pyrimidine oxyde-3 ou leurs sels d'addition d'acides.

**2.** Compsosition selon la revendication 1, caractérisée par le fait qu'elle se présente sous forme d'onguent, de teinture, de crème, de pommade, de poudre, de timbre, de tampon imbibé, de solution, d'émulsion, de dispersion vésiculaire, de lotion, de gel, de spray ou de suspension anhydre ou aqueuse, en vue de son application pharmaceutique et qu'elle contient au moins un composé tel que défini dans la revendication 1.

**3.** Composition pharmaceutique selon la revendication 2, caractérisé par le fait que le composé de formule (I) est présent à des concentrations comprises entre 0,1 et 10% en poids par rapport au poids total de la composition et en particulier entre 0,2 et 5% en poids.

**4.** Composition destinée à être utilisée en cosmétique, telle que définie dans la revendication 1, caractérisée par le fait qu'elle se présente sous forme de lotion, de gel, de savon, de shampooing, d'aérosol ou de mousse et qu'elle contient, dans un support acceptable sur le plan cosmétique, au moins un composé tel que défini dans la revendication 1 à une concentration comprise entre 0,01 et 5% en poids et en particulier entre 0,05 et 3% en poids.

**5.** Composition selon la revendication 4, caractérisée par le fait qu'elle contient à titre de composés améliorant encore l'activité sur la repousse et/ou le freinage de la chute des cheveux, des composés choisis parmi le Diazoxyde, la Spiroxasone, les phospholipides, les acides linoléique et linolénique, les acides hydroxycarboxyliques ou cétocarboxyliques, leurs esters, les lactones et leurs sels correspondants, l'anthraline, les caroténoïdes, les acides eicosatétraynoïque-5,8,11, 14 et eicosatriynoïque-5,8,11, leurs esters et amides, l'acide salicylique et ses dérivés porteurs d'un groupement alcanoyle ayant 2 à 12 atomes de carbone en position 5 du noyau benzenique.

**6.** Composition selon l'une quelconque des revendications 1 à 5, caractérisée par le fait qu'elle contient également des agents tensio-actifs choisis parmi les agents tensio-actifs non ioniques et amphotères.

**7.** Composition selon l'une quelconque des revendications 1 à 6, caractérisée par le fait que le milieu cosmétiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

**8.** Composition selon la revendication 7, caractérisée par le fait que les solvants sont choisis parmi les al-

cools inférieurs en $C_1$-$C_4$, les alkylène glycols et les alkyléthers de mono- et de dialkylène glycol.

**9.** Composition selon l'une quelconque des revendications 1 à 8, caractérisée par le fait que le milieu cosmétiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UVA et UVB, des agents antioxydants.

**10.** Composé, caractérisé par le fait qu'il répond à la formule :

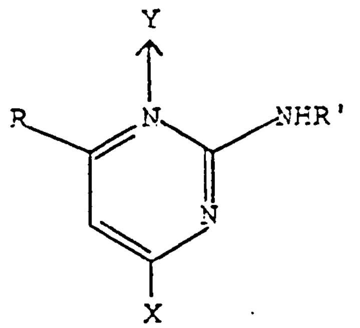

(I')

dans laquelle :

R désigne un atome d'hydrogène ou un radical alkyle en $C_1$-$C_8$ saturé, linéaire, ou forme avec le cycle pyrimidine, un cycle hydrocarboné de formule :

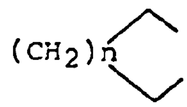

avec n égal à 1, 2 ou 3;

R' désigne un atome d'hydrogène ou l'un des radicaux suivants :

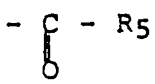 , 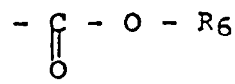 ou 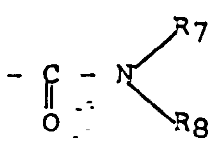

dans lesquels :

$R_5$ et $R_6$ désignent un radical alkyle inférieur en $C_1$-$C_4$;

$R_7$ et $R_8$ désignent un atome d'hydrogène ou un radical alkyle inférieur en $C_1$-$C_4$, à condition qu'ils ne désignent pas simultanément un atome d'hydrogène;

Y désigne un atome d'oxygène ou un groupement $-OSO_3^{\ominus}$;

X désigne :

(i) un groupement $SR_4$, dans lequel $R_4$ désigne un radical alkyle saturé en $C_1$-$C_{12}$, linéaire ou ramifié, pouvant être substitué par un atome d'halogène ou un radical trifluorométhyle, un radical alcényle linéaire en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un atome d'halogène, un radical alkyle en $C_1$-$C_6$, un radical alcoxy en $C_1$-$C_6$ ou un radical trifluorométhyle;

(ii) un groupement $-OR_3$, dans lequel $R_3$ a la même signification que $R_4$, à la condition que lorsque Y désigne un atome d'oxygène, R un groupement alkyle et R' désigne un atome d'hydrogène, $R_3$ ne désigne pas un radical alkyle en $C_1$-$C_4$ ou un groupement phényle éventuellement substitué par un ou deux atomes d'halogène ; ainsi que ses sels d'addition d'acides cosmétiquement ou pharmaceutiquement acceptables.

**11.** Composé selon la revendication 10, caractérisé par le fait qu'il est choisi parmi :

- l'amino-2 méthyl-4 (diméthyl-2,2)propyloxy-6 pyrimidine oxyde-3 ;
- l'amino-2 méthyl-4 (cyclohexyl-1) méthyloxy-6 pyrimidine oxyde-3 ;
- l'amino-2 méthyl-4 (hexenyl-5)oxy-6 pyrimidine oxyde-3 ;
- l'amino-2 méthyl-4 benzyloxy-6 pyrimidine oxyde-3 ;
- l'amino-2 méthyl-4 (diméthyl-2,4)phényloxy-6 pyrimidine oxyde-3 ;
- l'amino-2 méthyl-4 méthylthio-6 pyrimidine oxyde-3 ;
- l'amino-2 methyl-4 éthylthio-6 pyrimidine oxyde-3 ;
- le sel interne de l'hydroxyde de l'amino-2 méthyl-4 méthoxy-6 sulfooxy-3 pyrimidinium ;
- le sel interne de l'hydroxyde de l'amino-2 méthyl-4 éthoxy-6 sulfooxy-3 pyrimidinium ;
- l'amino-2 tétraméthylène-4,5 butoxy-6 pyrimidine oxyde-3 ;
- le sel interne de l'hydroxyde de l'amino-2 tétraméthylène-4,5 butoxy-6 sulfooxy-3 pyrimidinium.

**12.** Composé caractérisé par le fait qu'il répond à la formule suivante :

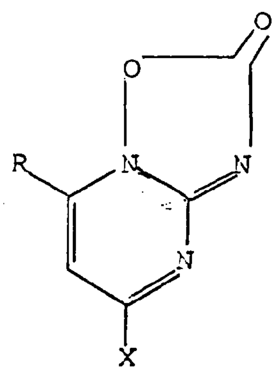

(III)

dans laquelle :

R désigne un atome d'hydrogène, un radical alkyle, saturé et linéaire en $C_1$-$C_8$ ou forme avec le cycle pyrimidine un cycle hydrocarboné de formule :

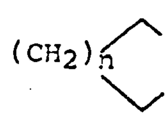

où n est égal à 1, 2 ou 3;

X désigne :

(i) un atome d'hydrogène;

(ii) un groupement

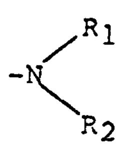

,

dans lequel

$R_1$ et $R_2$, identiques ou différents, désignent un atome d'hydrogène, un groupe alkyle saturé en $C_1$-$C_{12}$, linéaire ou ramifié, pouvant être substitué par un atome d'halogène ou un radical trifluorométhyle, un groupe alcényle linéaire en $C_2$-$C_{12}$, un groupe cycloalkyle en $C_3$-$C_{10}$, un groupe aralkyle en $C_7$-$C_{12}$ ou un groupenent aryle répondant à la formule :

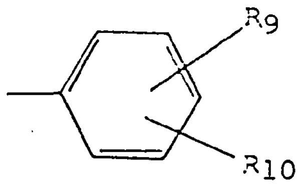

dans laquelle $R_9$ et $R_{10}$, identiques ou différentes, désignent hydrogène, alkyle en $C_1$-$C_4$, hydroxyle, alcoxy en $C_1$-$C_4$ ou halogène;

$R_1$ et $R_2$ peuvent former avec l'atome d'azote auquel ils sont reliés, un hétérocycle, saturé ou insaturé, choisi parmi : aziridino, azétidino, pyrrolidino, pipéridino, hexaméthylèneimino, heptaméthylèneimino, octaméthylèneimino, tétrahydro pyridino, dihydropyridino, pyrrole, pyrazole, imidazole, thiomorpholino;

(iii) un groupement $-OR_3$, dans lequel :

$R_3$ désigne un radical alkyle saturé en $C_1$-$C_{12}$, linéaire ou ramifié, pouvant être substitué par un atome d'halogène ou un radical trifluorométhyle, un radical alcényle linéaire en $C_2$-$C_{12}$, un radical cycloalkyle en $C_3$-$C_{10}$, un radical aralkyle en $C_7$-$C_{12}$, un radical phényle éventuellement substitué par un ou deux groupements qui, indépendamment l'un de l'autre, désignent un atome d'halogène, un radical alkyle en $C_1$-$C_6$, un radical alcoxy en $C_1$-$C_6$, ou un radical trifluorométhyle;

(iv) un groupement $-SR_4$, dans lequel $R_4$ a la même signification que $R_3$;

à la condition qu'il soit différent des composés 2-oxo-2,8-dihydro[1,2,4]oxadiazolo[2,3-a]pyrimidine carbamate et 2H-[1,2,4]oxadiazolo [2,3-a] pyrimidine ou l'un de ses sels d'addition d'acides physiologiquement acceptables.

**13.** Composition cosmétique pour freiner la chute des cheveux et pour induire et stimuler leur croissance, caractérisée par le fait qu'elle contient dans un milieu cosmétiquement acceptable, au moins un composé répondant à la formule (III) définie dans la revendication 12.

**14.** Utilisation des composés répondant à la formule générale (I) :

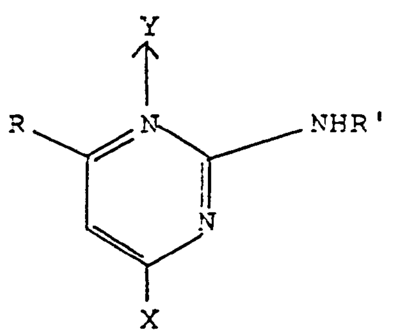

(I)

dans laquelle R, R', X et Y ont la signification indiquée dans la revendication 1, ou de leurs sels d'addition d'acides pharmaceutiquement acceptables, pour la préparation d'un médicament destiné au traitement thérapeutique de l'alopécie, de la pelade, de la chute des cheveux et des dermatites desquamantes.

**15.** Procédé de traitement cosmétique des cheveux ou du cuir chevelu, caractérisé par le fait que l'on applique la composition telle que définie dans les revendications 1 à 9 et 13.

**16.** Utilisation des composés de formule (III) selon la revendication 12, ou l'un de ses sels d'addition d'acide physiologiquement acceptable, pour la préparation d'un médicament pour traiter l'alopécie, la pelade, la chute des cheveux et les dermatites desquamantes.

**17.** Procédé de préparation d'une composition pharmaceutique destinée à freiner la chute des cheveux, induire et stimuler leur croissance, caractérisé par le fait que l'on incorpore 0,1 et 10% en poids d'un composé de formule (I) selon la revendication 1, ou l'un de ses sels d'addition d'acides pharmaceutiquement acceptables, par rapport au poids total de la composition, dans un milieu pharmaceutiquement acceptable.

**18.** Procédé selon la revendication 17, caractérisé par le fait que la composition se présente sous forme d'onguent, de teinture, de crème, de pommade, de poudre, de timbre, de tampon imbibé, de solution, d'émulsion, de dispersion vésiculaire, de lotion, de gel, de spray, de suspension anhydre ou aqueuse.

## Patentansprüche

## Patentansprüche für folgende Vertragsstaaten : AT, BE, CH, DE, DK, FR, GB, IT, LI, NL, SE

**1.** Zusammensetzung zur Verwendung zur Verminderung des Haarsausfalls und zum Induzieren und Stimulieren des Haarwuchses, dadurch gekennzeichnet, daß sie in einem kosmetisch annehmbaren Milieu mindestens eine Verbindung der folgenden Formel enthält:

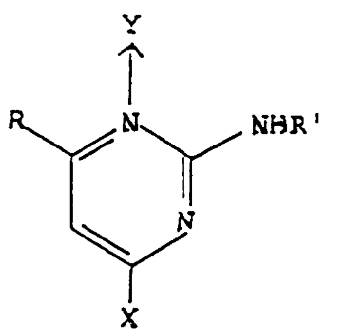

(I)

worin bedeuten:
R ein Wasserstoffatom, ein gesättigtes und lineares $C_1$-$C_8$-Alkylradikal, oder R mit dem Pyrimidinring einen Kohlenstoffring der Formel:

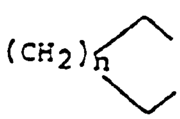

bilden kann worin n gleich 1, 2 oder 3 ist;
X bedeutet:
(i) ein Wasserstoffatom;
(ii) eine Gruppe

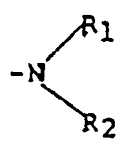

worin $R_1$ und $R_2$ identisch oder verschieden sind und ein Wasserstoffatom, eine gesättigte, lineare oder verzweigte $C_1$-$C_{12}$-Alkylgruppe bedeuten, die durch ein Halogenatom oder ein Trifluormethylradikal substituiert sein kann, eine lineare $C_2$-$C_{12}$-Alkenylgruppe, eine $C_3$-$C_{10}$-Cykloalkylgruppe, eine $C_7$-$C_{12}$-Aralkylgruppe oder eine Arylgruppe der Formel:

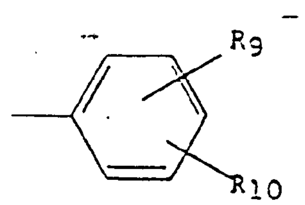

worin
R9 und R10 gleich oder verschieden sein können und ein Wasserstoffatom, C1-C4-Alkyl, Hydroxyl, C1-C4-Alkoxy oder Halogen bedeuten;
R1 und R2 zusammen mit dem Stickstoffatom, an das sie gebunden sind, einen gesättigten oder ungesättigten Heterozyklus bilden können, ausgewählt aus: Aziridino, Azetidino, Pyrrolidino, Piperidino, Hexamethylenimino, Heptamethylenimino, Octamethylenimino, Tetrahydropyridino, Dihydropyridino, Pyrrol, Pyrazol, Imidazol, Triazol, 4-Alkyl-Piperazino, Morpholino, Thiomorpholino;
(iii) eine Gruppe -OR3 worin bedeutet:
R3 ein gesättigtes lineares oder verzweigtes C1-C12-Alkylradikal, das durch ein Halogenatom oder ein Trifluormethylradikal substituiert sein kann, ein lineares C2-C12-Alkenylradikal, ein C3-C10-Cykloalkylradikal, ein C7-C12-Aralkylradikal, ein gegebenenfalls durch 1 oder 2 Gruppen, die unabhängig voneinander ein Halogenatom, ein C1-C6-Alkylradikal, ein C1-C6-Alkoxyradikal oder ein Trifluormethylradikal bedeuten können, substituiertes Phenylradikal;
(iv) eine Gruppe -SR4, worin R4 die gleiche Bedeutung wie R3 besitzt;
Y ein Sauerstoffatom oder die Gruppe -OSO3- bedeutet;
R' ein Wasserstoffatom oder eines der folgenden Radikale bedeutet:

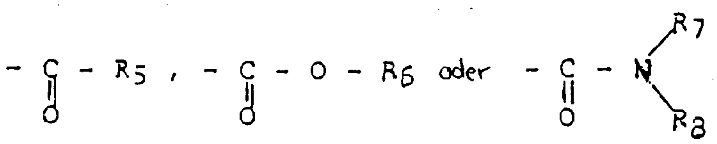

worin bedeuten:
R5 und R6 ein C1-C4-Niederalkylradikal;
R7 und R8 ein Wasserstoffatom oder ein C1-C4-Niederalkylradikal, mit der Maßgabe, daß keiner der Reste gleichzeitig ein Wasserstoffatom bedeutet;
oder ihre physiologisch annehmbaren Säureadditionssalze, mit der Maßgabe, daß, wenn R' Wasserstoff bedeutet, X nicht -NR1R2 bedeutet, und die Verbindung der Formel (I) von 2-Amino-pyrimidin-3-oxid oder 2-(Ethoxycarbonylamino)-pyrimidin-3-oxid oder ihren Säureadditionssalzen verschieden ist.

2. Zusammensetzung nach Anspruch 1, dadurch gekennzeichnet, daß sie in Form einer Salbe, Tinktur, Creme, Pomade, eines Pulvers, Pflasters, getränkten Tampons, einer Lösung, einer Emulsion, einer bläschenförmigen Dispersion, einer Lotion, eines Gels, eines Sprays oder einer wasserfreien oder wässerigen Suspension vorliegt, die für eine pharmazeutische Applikation geeignet ist, und die mindestens eine Verbindung, wie sie im Anspruch 1 definiert ist, enthält.

3. Pharmazeutische Zusammensetzung nach Anspruch 2, dadurch gekennzeichnet, daß die Verbindung der Formel (I) in Konzentrationen zwischen 0,1 und 10 Gew.-%, bezogen auf das Gesamtgewicht der Zusammensetzung, vorliegt, und insbesondere zwischen 0,2 und 5 Gew.-%.

4. Zusammensetzung zur kosmetischen Verwendung, wie sie im Anspruch 1 definiert ist, dadurch gekennzeichnet, daß sie in Form einer Lotion, eines Gels, einer Seife, eines Shampoos, eines Aerosols oder eines Schaums vorliegt, und in einem zur kosmetischen Verwendung geeigneten Träger mindestens eine Verbindung, wie sie im Anspruch 1 definiert ist, enthält.

5. Zusammensetzung nach Anspruch 4, dadurch gekennzeichnet, daß sie als Verbindungen, die die Wirkung der Abwehr und/oder der Verminderung des Haarausfalles verbessern, solche enthält,die ausgewählt sind unter Diazoxyd, Spiroxason, Phosphorlipiden, Linolsäuren und Linolensäuren, den Hydroxycarbonsäuren oder Ketocarbonsäuren, ihren Estern, den Lactonen und ihren entsprechenden Salzen,

Anthralin, den Carotinoiden, den Eicosatetrain-5,8,11,14-säuren und Eicosatriin-5,8,11-säuren, ihren Estern und Amiden, der Salicylsäure und ihren Derivaten, die eine Alkanoylgruppe mit 2 bis 12 Kohlenstoffatomen in 5 Stellung des Benzolringes tragen.

**6.** Zusammensetzung nach einem der Ansprüche 1 bis 5, dadurch gekennzeichnet, daß sie außerdem oberflächenaktive Mittel,ausgewählt aus den nicht-ionischen und amphoteren oberflächenaktiven Mitteln, enthält.

**7.** Zusammensetzung nach einem der Ansprüche 1 bis 6, dadurch gekennzeichnet, daß das kosmetisch annehmbare Milieu aus Wasser, einer Mischung aus Wasser und einem oder mehreren organischen Lösungsmitteln, oder aus einer Mischung von organischen Lösungsmitteln besteht, wobei die organischen Lösungsmittel pharmazeutisch oder kosmetisch annehmbar sind.

**8.** Zusammensetzung nach Anspruch 7, dadurch gekennzeichnet, daß die Lösungsmittel ausgewählt sind unter den C1-C4-Niederalkoholen, den Alkylenglycolen und den Alkylethern von Mono- und Dialkylenglycol.

**9.** Zusammensetzung nach einem der Ansprüche 1 bis 8, dadurch gekennzeichnet, daß das kosmetisch annehmbare Milieu mittels Verdickungsmitteln und/oder Geliermitteln verdickt ist und Konservierungsmittel, Stabilisatoren, pH-Regulatoren, Modifiziermittel für den osmotischen Druck, Emulgiermittel, UVA- und UVB-Filter und Antioxydantien enthält.

**10.** Verbindung, dadurch gekennzeichnet, daß sie der Formel entspricht:

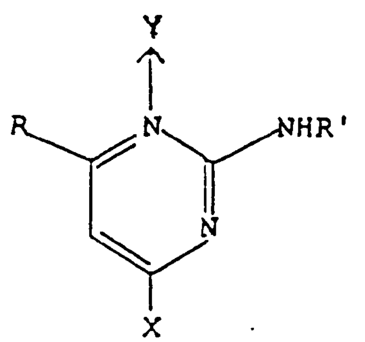

(I')

worin bedeuten:
R ein Wasserstoffatom oder ein gesättigtes lineares C1-C8-Alkylradikal, oder R zusammen mit dem Pyrimidinring einen Kohlenstoffring der Formel bildet:

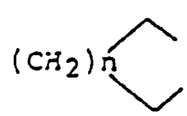

worin n gleich 1, 2 oder 3 ist;
R' ein Wasserstoffatom oder eines der folgenden Radikale bedeutet:

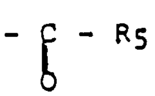 , 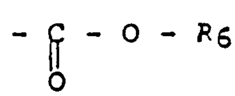 oder 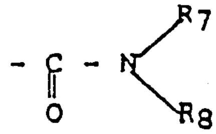

worin bedeuten:
R5 und R6 ein C1-C4-Niederalkylradikal;
R7 und R8 ein Wasserstoffatom oder ein C1-C4-Niederalkylradikal bedeuten, mit der Maßgabe, daß Sie nicht gleichzeitig ein Wasserstoffatom bedeuten;

Y ein Sauerstoffatom oder die Gruppe -OSO3⁻ bedeutet;
X bedeutet:

(i) eine Gruppe SR4, worin R4 ein gesättigtes lineares oder verzweigtes C1-C12-Alkylradikal bedeutet, das durch ein Halogenatom oder ein Trifluormethylradikal ersetzt sein kann, ein lineares C2-C12-Alkenylradikal, ein C3-C10-Cykloalkylradikal, ein C7-C12-Aralkylradikal, ein gegebenenfalls durch 1 oder 2 Gruppen substituiertes Phenylradikal, die unabhängig voneinander ein Halogenatom, ein C1-C6-Alkylradikal, ein C1-C6-Alkoxyradikal oder ein Trifluormethylradikal bedeuten;
(ii) eine Gruppe -OR3, worin R3 die gleiche Bedeutung wie R4 besitzt, unter der Bedingung, daß, wenn Y ein Sauerstoffatom, R eine Alkylgruppe und R' ein Wasserstoffatom bedeutet, R3 kein C1-C4 Alkylradikal und keine gegebenenfalls durch 1 oder 2 Halogenatome substituierte Gruppe bedeutet; sowie die kosmetisch oder pharmazeutisch annehmbaren Säureadditionssalze.

**11.** Verbindung nach Anspruch 10, dadurch gekennzeichnet, daß sie ausgewählt ist aus den folgenden:

- 2-Amino-4-methyl-6-(2,2-dimethylpropyloxy)-pyrimidin-3-oxid;
- 2-Amino-4-methyl-6-(1-cyclohexylmethyloxy)-pyrimidin-3-oxid;
- 2-Amino-4-methyl-6-(hexen-5-yloxy)-pyrimidin-3-oxid;
- 2-Amino-4-methyl-6-benzyloxy-pyrimidin-3-oxid;
- 2-Amino-4-methyl-6-(2,4-dimethyl-phenyloxy)-pyrimidin-3-oxid;
- 2-Amino-4-methyl-6-methylthio-pyrimidin-3-oxid;
- 2-Amino-4-methyl-6-ethylthio-pyrimidin-3-oxid;
- internes Salz von 2-Amino-4-methyl-6-methoxy-3-sulfooxy-pyrimidiniumhydroxid;
- internes Salz von 2-Amino-4-methyl-6-ethoxy-3-sulfooxy-pyrimidiniumhydroxid:
- 2-Amino-4,5-tetramethylen-6-butoxy-pyrimidin-3-oxid;
- internes Salz von 2-Amino-4,5-tetramethylen-6-butoxy-3-sulfooxy-pyrimidiniumhydroxid.

**12.** Verbindung gekennzeichnet dadurch, daß sie der folgenden Formel entspricht:

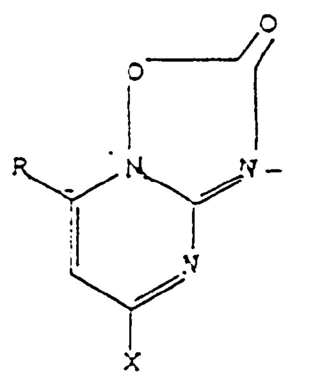

(III)

worin bedeuten:
R ein Wasserstoffatom, ein gesättigtes und lineares C1-C8-Alkylradikal, oder R zusammen mit dem Pyrimidinring einen Kohlenstoffring der Formel bildet,

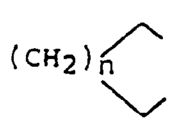

worin n 1, 2 oder 3 bedeutet;
X bedeutet:

(i) ein Wasserstoffatom
(ii) eine Gruppe

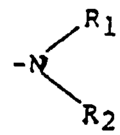

worin R1 und R2 identisch oder verschieden sind und ein Wasserstoffatom, eine gesättigte lineare oder verzweigte C1-C12-Alkylgruppe bedeuten, die durch ein Halogenatom oder ein Trifluormethylradikal substituiert sein kann, eine lineare C2-C12-Alkenylgruppe, eine C3-C10-Cycloalkylgruppe, eine C7-C12-Aralkylgruppe oder eine Arylgruppe der Formel:

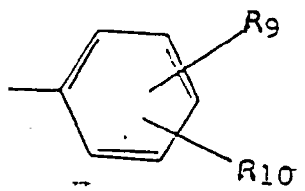

worin R9 und R10 identisch oder verschieden sein können und Wasserstoff, C1-C4-Alkyl, Hydroxyl, C1-C4-Alkoxy oder Halogen bedeuten;
R1 und R2 mit dem Stickstoffatom, an das sie gebunden sind, einen gesättigten oder ungesättigten Heterocyclus bilden können, der ausgewählt ist aus: Aziridino, Azetidino, Pyrrolidino, Piperidino, Hexamethylenimino, Heptamethylenimino, Octamethylenimino, Tetrahydropyridino, Dihydropyridino, Pyrrol, Pyrazol, Imidazol, Thiomorpholino,
(iii) eine Gruppe -OR3, worin bedeuten:
R3 ein gesättigtes lineares oder verzweigtes C1-C12-Alkylradikal, das durch ein Halogenatom oder ein Trifluormethylradikal substituiert sein kann, ein lineares C2-C12-Alkenylradikal, ein C3-C10-Cycloalkylradikal, ein C7-C12-Aralkylradikal, ein gegebenenfalls durch 1 oder 2 Gruppen substituiertes Phenylradikal, die unabhängig voneinander ein Halogenatom, ein C1-C6-Alkylradikal, ein C1-C6-Alkoxyradikal oder ein Trifluormethylradikal sein können;
(iv) eine Gruppe -SR4, worin R4 die gleiche Bedeutung wie R3 besitzt;
mit der Maßgabe, daß sie verschieden ist von den Verbindungen 2-Oxo-2,8-dihydro[1,2,4]oxadiazolo[2,3-a]pyrimidincarbamat und 2H-[1,2,4]oxadiazolo[2,3-a]pyrimidin oder einem ihrer physiologisch annehmbaren Säureadditionssalze.

**13.** Kosmetische Zusammensetzung zur Verminderung des Haarausfalles und zur Induzierung und Stimulierung des Haarwuchses, dadurch gekennzeichnet, daß sie in einem kosmetisch annehmbaren Milieu mindestens eine Verbindung der im Anspruch 12 definierten Formel (III) enthält.

**14.** Verwendung der Verbindungen der allgemeinen Formel (I):

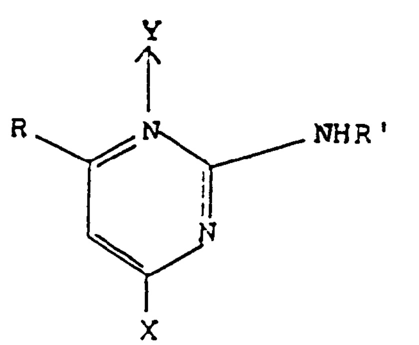

(I)

worin R, R', X und Y die im Anspruch 1 angegebenen Bedeutungen besitzen, oder ihrer pharmazeutisch annehmbaren Säureadditionssalze zur Herstellung eines Medikaments zur therapeutischen Behandlung der Alopexie, von Haarschwund, von Haarausfall und von Schuppen.

**15.** Verfahren zur kosmetischen Behandlung der Haare oder der Kopfhaut, dadurch gekennzeichnet, daß man

eine Zusammensetzung nach einem der Ansprüche 1 bis 9 und 13 appliziert.

16. Verwendung der Verbindung der Formel (III) gemäß Anspruch 12 oder eines ihrer physiologisch annehmbaren säureadditionssalze zur Herstellung eines Medikaments zur Behandlung der Alopexie, von Haarschwund, von Haarausfall und von Schuppen.

17. Verfahren zur Herstellung einer pharmazeutischen Zusammensetzung zur Verminderung des Haarausfalles, zur Induzierung und zur Stimulierung des Haarwuchses, dadurch gekennzeichnet, daß man 0,1 bis 10 Gew.-% einer Verbindung der Formel (I) gemäß Anspruch 1, oder eines ihrer pharmazeutisch annehmbaren Säureadditionssalze, bezogen auf das Gesamtgewicht der Zusammensetzung, in ein pharmazeutisch annehmbares Milieu einbringt.

18. Verfahren nach Anspruch 17, dadurch gekennzeichnet, daß die Zusammensetzung in Form einer Salbe, einer Tinktur, einer Creme, einer Pomade, eines Pulvers, eines Pflasters, eines getränkten Tampons, einer Lösung, einer Emulsion, einer bläschenförmigen Dispersion, einer Lotion, eines Gels, eines Sprays oder einer wasserfreien oder wässerigen Suspension vorliegt.

## Claims

**Claims for the following Contracting States : AT, BE, CH, DE, DK, FR, GB, IT, LI, NL, SE**

1. Composition intended for use for retarding hair loss and for inducing and stimulating its growth, characterized in that it contains, in a cosmetically acceptable medium, at least one compound corresponding to the following formula:

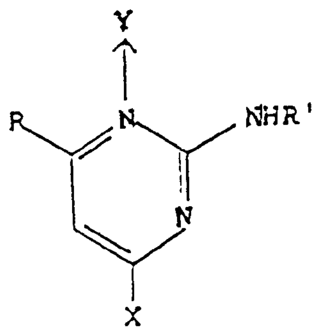

(I)

in which:

R denotes a hydrogen atom or a saturated linear $C_1$-$C_8$ alkyl radical or, with the pyrimidine ring, forms a carbon/hydrogen-containing ring of formula:

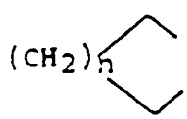

where n is equal to 1, 2 or 3;

X denotes:

(i) a hydrogen atom;

(ii) a group

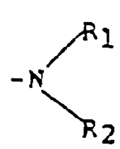

,

in which:

$R_1$ and $R_2$, which may be identical or different, denote a hydrogen atom, a saturated linear or branched $C_1$-$C_{12}$ alkyl group which can be substituted with a halogen atom or a trifluoromethyl radical, a linear $C_2$-$C_{12}$ alkenyl group, a $C_3$-$C_{10}$ cycloalkyl group, a $C_7$-$C_{12}$ aralkyl group or an aryl group corresponding to the formula:

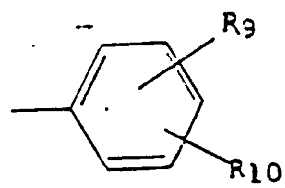

in which $R_9$ and $R_{10}$, which may be identical or different, denote hydrogen, $C_1$-$C_4$ alkyl, hydroxyl, $C_1$-$C_4$ alkoxy or halogen;

$R_1$ and $R_2$, with the nitrogen atom to which they are attached, can form a saturated or unsaturated heterocycle selected from aziridino, azetidino, pyrrolidino, piperidino, hexamethylenimino, heptamethylenimino, octamethylenimino, tetrahydropyridino, dihydropyridino, pyrrole, pyrrazole, imidazole, triazole, 4-alkylpiperazino, morpholino, thiomorpholino;

(iii) a group $-OR_3$, in which:

$R_3$ denotes a saturated linear or branched $C_1$-$C_{12}$ alkyl radical which can be substituted with a halogen atom or a trifluoromethyl radical, a linear $C_2$-$C_{12}$ alkenyl radical, a $C_3$-$C_{10}$ cycloalkyl radical, a $C_7$-$C_{12}$ aralkyl radical or a phenyl radical optionally substituted with one or two groups which, independently of one another, denote a halogen atom, a $C_1$-$C_6$ alkyl radical, a $C_1$-$C_6$ alkoxy radical, or a trifluoromethyl radical;

(iv) a group $-SR_4$ in which $R_4$ has the same meaning as $R_3$;

Y denotes an oxygen atom or an $-OSO_3^{\ominus}$ group; and

R' denotes a hydrogen atom or one of the following radicals:

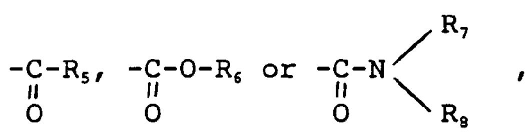

in which

$R_5$ and $R_6$ represent $C_1$-$C_4$ lower alkyl radicals; and

$R_7$ and $R_8$ denote a hydrogen atom or a $C_1$-$C_4$ lower alkyl radical, on condition that they do not simultaneously denote a hydrogen atom;

or its addition salts with physiologically acceptable acids, with the proviso that, when R' denotes hydrogen, X does not denote $-NR_1R_2$, and that the compound of formula (I) is different from 2-aminopyrimidine 3-oxide or 2-(ethoxycarbonylamino)pyrimidine 3-oxide or their addition salts with acids.

2. Composition according to Claim 1, characterized in that it is presented in the form of an ointment, tincture, cream, pomade, powder, patch, impregnated pad, solution, emulsion, vesicular dispersion, lotion, gel, spray or anhydrous or aqueous suspension, for the purpose of its pharmaceutical application and in that it contains at least one compound as defined in Claim 1.

3. Pharmaceutical composition according to Claim 2, characterized in that the compound of formula (I) is present at concentrations of between 0.1 and 10% by weight relative to the total weight of the composition, and especially between 0.2 and 5% by weight.

4. Composition intended for use in cosmetics, as defined in Claim 1, characterized in that it is presented in the form of a lotion, gel, soap, shampoo, aerosol or foam, and in that it contains, in a vehicle acceptable from a cosmetic standpoint, at least one compound (lacuna)

5. Composition according to Claim 4, characterized in that it contains, by way of compounds further improving the activity with respect to hair regrowth and/or to retarding its loss, compounds selected from diazoxide, spiroxasone, phospholipids, linoleic and linolenic acids, hydroxy-carboxylic or keto-carboxylic acids, their esters and lactones and their corresponding salts, anthralin, carotenoids, 5,8,11,14-eicosatetraynoic and 5,8,11-eicosatriynoic acids, their esters and amides, and salicylic acid and its derivatives bearing an alkanoyl group having 2 to 12 carbon atoms at the 5-position of the benzene ring.

**6.** Composition according to any one of Claims 1 to 5, characterized in that it also contains surfactants selected from nonionic and amphoteric surfactants.

**7.** Composition according to any one of Claims 1 to 6, characterized in that the cosmetically acceptable medium consists of water or a mixture of water and one or more organic solvent(s), or of a mixture of organic solvents, the organic solvents being pharmaceutically or cosmetically acceptable.

**8.** Composition according to Claim 7, characterized in that the solvents are selected from $C_1$-$C_4$ lower alcohols, alkylene glycols and mono- and dialkylene glycol alkyl ethers.

**9.** Composition according to any one of Claims 1 to 8, characterized in that the cosmetically acceptable medium is thickened by means of thickening and/or gelling agents, and contains preservatives, stabilizers, pH regulators, osmotic pressure-modifying agents, emulsifiers, UV-A and UV-B screening agents, antioxidants.

**10.** Compound characterized in that it corresponds to the formula:

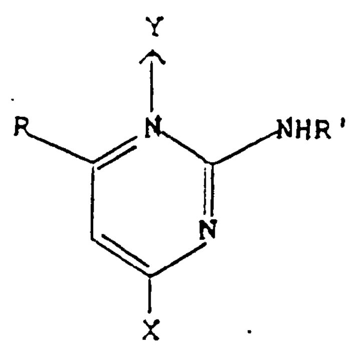

(I′)

in which:

R denotes a hydrogen atom or a saturated linear $C_1$-$C_8$ alkyl radical or, with the pyrimidine ring, forms a carbon/hydrogen-containing ring of formula:

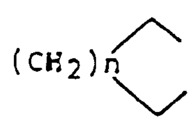

with n equal to 1, 2 or 3;

R' denotes a hydrogen atom or one of the following radicals:

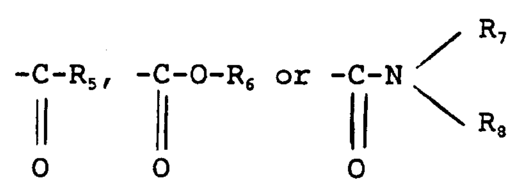

in which:

$R_5$ and $R_6$ denote a $C_1$-$C_4$ lower alkyl radical; and

$R_7$ and $R_8$ denote a hydrogen atom or a $C_1$-$C_4$ lower alkyl radical, on condition that they do not simultaneously denote a hydrogen atom;

Y denotes an oxygen atom or an $-OSO_3^{\ominus}$ group; and

X denotes:

(i) a group $SR_4$, in which $R_4$ denotes a saturated linear or branched $C_1$-$C_{12}$ alkyl radical which can be substituted with a halogen atom or a trifluoromethyl radical, a linear $C_2$-$C_{12}$ alkenyl radical, a $C_3$-$C_{10}$ cycloalkyl radical, a $C_7$-$C_{12}$ aralkyl radical or a phenyl radical optionally substituted with one or two groups which, independently of one another, denote a halogen atom, a $C_1$-$C_6$ alkyl radical, a $C_1$-$C_6$ alkoxy radical or a trifluoromethyl radical;

(ii) a group $-OR_3$ in which $R_3$ has the same meaning as $R_4$, on condition that, when Y denotes an oxygen atom, R an alkyl group and R' denotes a hydrogen atom, $R_3$ does not denote a $C_1$-$C_4$ alkyl radical or a phenyl group optionally substituted with one or two halogen atoms; as well as its addition salts with cosmetically or pharmaceutically acceptable acids.

**11.** Compound according to Claim 10, characterized in that it is selected from:

- 2-amino-4-methyl-6-(2,2-dimethylpropyloxy)pyrimidine 3-oxide;
- 2-amino-4-methyl-6-(1-cyclohexylmethyloxy)pyrimidine 3-oxide;
- 2-amino-4-methyl-6-(5-hexenyloxy)pyrimidine 3-oxide;
- 2-amino-4-methyl-6-benzyloxypyrimidine 3-oxide;
- 2-amino-4-methyl-6-(2,4-dimethylphenyloxy)pyrimidine 3-oxide;
- 2-amino-4-methyl-6-methylthiopyrimidine 3-oxide;
- 2-amino-4-methyl-6-ethylthiopyrimidine 3-oxide;
- the internal salt of 2-amino-4-methyl-6-methoxy-3-sulphooxypyrimidinium hydroxide;
- the internal salt of 2-amino-4-methyl-6-ethoxy-3-sulphooxypyrimidinium hydroxide;
- 2-amino-4 , 5-tetramethylene-6-butoxypyrimidine 3-oxide; -

the internal salt of 2-amino-4,5-tetramethylene-6-butoxy-3-sulphooxypyrimidinium hydroxide.

**12.** Compound characterized in that it corresponds to the following formula:

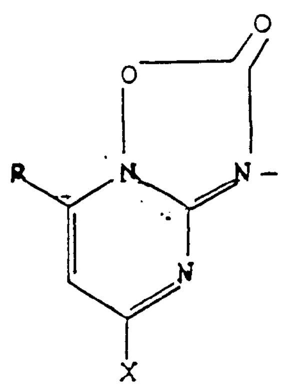

(III)

in which:

R denotes a hydrogen atom or a saturated linear $C_1$-$C_8$ alkyl radical or, with the pyrimidine ring, forms a carbon/hydrogen-containing ring of formula:

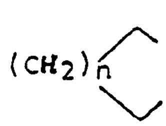

where n is equal to 1, 2 or 3; and

X denotes:

(i) a hydrogen atom;

(ii) a group

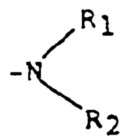

,

in which:

$R_1$ and $R_2$, which may be identical or different, denote a hydrogen atom, a saturated linear or branched $C_1$-$C_{12}$ alkyl group which can be substituted with a halogen atom or a trifluoromethyl radical, a linear $C_2$-$C_{12}$ alkenyl group, a $C_3$-$C_{10}$ cycloalkyl group, a $C_7$-$C_{12}$ aralkyl group or an aryl group corresponding to the formula:

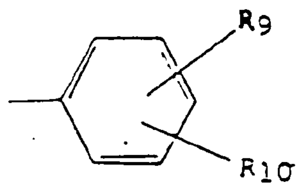

in which $R_9$ and $R_{10}$, which may be identical or different, denote hydrogen, $C_1$-$C_4$ alkyl, hydroxyl, $C_1$-$C_4$ alkoxy or halogen;

$R_1$ and $R_2$, with the nitrogen atom to which they are attached, can form a saturated or unsaturated heterocycle selected from aziridino, azetidino, pyrrolidino, piperidino, hexamethylenimino, heptamethylenimino, octamethylenimino, tetrahydropyridino, dihydropyridino, pyrrole, pyrazole, imidazole, thiomorpholino;

(iii) a group $-OR_3$, in which:

$R_3$ denotes a saturated linear or branched $C_1$-$C_{12}$ alkyl radical which can be substituted with a halogen atom or a trifluoromethyl radical, a linear $C_2$-$C_{12}$ alkenyl radical, a $C_3$-$C_{10}$ cycloalkyl radical, a $C_7$-$C_{12}$ aralkyl radical or a phenyl radical optionally substituted with one or two groups which, independently of one another, denote a halogen atom, a $C_1$-$C_6$ alkyl radical, a $C_1$-$C_6$ alkoxy radical, or a trifluoromethyl radical;

(iv) a group $-SR_4$, in which $R_4$ has the same meaning as $R_3$;

on condition that it is different from the compounds 2-oxo-2,8-dihydro-[1,2,4]oxadiazolo[2,3-a]pyrimidine carbamate and 2H-[1,2,4]oxadiazolo[2,3-a]pyrimidine, or one of its addition salts with physiologically acceptable acids.

**13.** Cosmetic composition for retarding hair loss and for inducing and stimulating its growth, characterized in that it contains, in a cosmetically acceptable medium, at least one compound corresponding to the formula (III) defined in Claim 12.

**14.** Use of the compounds corresponding to the general formula (I):

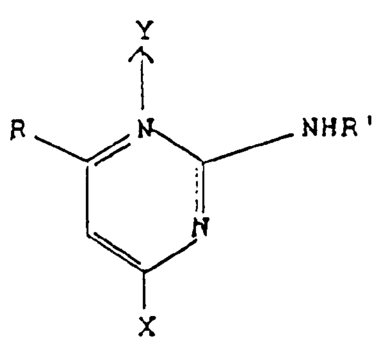

in which R, R', X and Y have the meaning stated in Claim 1, or their addition salts with pharmaceutically acceptable acids, for the preparation of a medicinal product intended for the therapeutic treatment of alopecia, pelade, hair loss and desquamating dermatitis.

**15.** Process for cosmetic treatment of the hair or scalp, characterized in that the composition as defined in Claims 1 to 9 and 13 is applied.

**16.** Use of the compounds of the formula (III) according to Claim 12, or one of its addition salts with a physiologically acceptable acid, for the preparation of a medicinal product for treating alopecia, pelade, hair loss and desquamating dermatitis.

**17.** Process for preparing a pharmaceutical composition intended for retarding hair loss and inducing and stimulating its growth, characterized in that 0.1 and (sic) 10% by weight of a compound of formula (I) according to Claim 1, or one of its addition salts with pharmaceutically acceptable acids, relative to the total weight of the composition, is incorporated in a pharmaceutically acceptable medium.

**18.** Process according to Claim 17, characterized in that the composition is presented in the form of an ointment, tincture, cream, pomade, powder, patch, impregnated pad, solution, emulsion, vesicular dispersion, lotion, gel, spray or anhydrous or aqueous suspension.